# EUROPEAN PATENT SPECIFICATION

(11) **EP 2 694 112 B1**
(45) Date of publication and mention of the grant of the patent: **08.11.2017**
(21) Application number: 12723769.1
(22) Date of filing: 06.04.2012
(51) Int. Cl.: C07K 14/715, C07K 14/705, C07K 16/28, C12N 15/62, G01N 33/574, A61K 39/00

(54) **ANTI-TUMOR ANTIBODY-TUMOR SUPPRESSOR FUSION PROTEIN COMPOSITIONS AND METHODS OF USE FOR THE TREATMENT OF CANCER**
ANTITUMOR- UND ANTIKÖRPERTUMOR-HEMMER-FUSIONSPROTEIN-ZUSAMMENSETZUNGEN UND ANWENDUNGSVERFAHREN DAFÜR ZUR BEHANDLUNG VON KREBS
COMPOSITIONS DE PROTÉINE DE FUSION ANTICORPS ANTITUMORAL-SUPPRESSEUR DE TUMEUR ET PROCÉDÉ D'UTILISATION POUR LE TRAITEMENT DU CANCER

(30) Priority: 07.04.2011 US 201161516738 P
(43) Date of publication of application: 12.02.2014
(73) Proprietor: Memorial Sloan-Kettering Cancer Center, New York, NY 10065 (US)
(72) Inventor: WENDEL, Hans-Guido, New York NY 10023 (US); ORCCHIO, Elisa, NewYork NY 10028 (US)
(74) Representative: Pearl Cohen Zedek Latzer Baratz UK LLP
(86) International application number: PCT/US2012/032527
(87) International publication number: WO 2012/139002

(56) References cited:
- WO-A2-2009/150513
- DAWSON D W ET AL: "Global DNA methylation profiling reveals silencing of a secreted form of Epha7 in mouse and human germinal center B-cell lymphomas", ONCOGENE, vol. 26, no. 29, June 2007 (2007-06), pages 4243-4252, XP002684411, ISSN: 0950-9232
- XUE WEN ET AL: "Senescence and tumour clearance is triggered by p53 restoration in murine liver carcinomas", NATURE: INTERNATIONAL WEEKLY JOURNAL OF SCIENCE, NATURE PUBLISHING GROUP, UNITED KINGDOM, vol. 445, no. 7128, 8 February 2007 (2007-02-08), pages 656-660, XP002491252, ISSN: 0028-0836, DOI: 10.1038/NATURE05529
- SURAWSKA H ET AL: "The role of ephrins and Eph receptors in cancer", CYTOKINE AND GROWTH FACTOR REVIEWS, ELSEVIER LTD, GB, vol. 15, no. 6, 1 December 2004 (2004-12-01), pages 419-433, XP004652022, ISSN: 1359-6101, DOI: 10.1016/J.CYTOGFR.2004.09.002
- RELANDER THOMAS ET AL: "Prognostic factors in follicular lymphoma", JOURNAL OF CLINICAL ONCOLOGY,, vol. 28, no. 17, 10 June 2010 (2010-06-10) , pages 2902-2913, XP009162785, ISSN: 1527-7755
- NANJANGUD G ET AL: "Molecular cytogenetic analysis of follicular lymphoma (FL) provides detailed characterization of chromosomal instability associated with the t(14;18)(q32;q21) positive and negative subsets and histologic progression", CYTOGENETIC AND GENOME RESEARCH, ALLERTON PRESS, NEW YORK, NY, US, vol. 118, no. 2-4, 1 January 2007 (2007-01-01), pages 337-344, XP009162708, ISSN: 1424-8581, DOI: 10.1159/000108318
- ELISA ORICCHIO ET AL: "The Eph-Receptor A7 Is a Soluble Tumor Suppressor for Follicular Lymphoma", CELL, CELL PRESS, US, vol. 147, no. 3, 21 September 2011 (2011-09-21), pages 554-564, XP028330381, ISSN: 0092-8674, DOI: 10.1016/J.CELL.2011.09.035 [retrieved on 2011-09-28]
- TSUBOI MASARU ET AL: "Secreted form of EphA7 in lung cancer", INTERNATIONAL JOURNAL OF ONCOLOGY, DEMETRIOS A. SPANDIDOS ED. & PUB, GR, vol. 36, no. 3, 1 March 2010 (2010-03-01), pages 635-640, XP009162702, ISSN: 1019-6439, DOI: 10.3892/IJO_00000539
- WANG LIN-FANG ET AL: "Increased expression of EphA7 correlates with adverse outcome in primary and recurrent glioblastoma multiforme patients", BMC CANCER, BIOMED CENTRAL, LONDON, GB, vol. 8, no. 1, 25 March 2008 (2008-03-25), page 79, XP021034714, ISSN: 1471-2407
- HANSEN ET AL: "Antibody-mediated p53 protein therapy prevents liver metastasis in vivo", CANCER RESEARCH, AMERICAN ASSOCIATION FOR CANCER RESEARCH, US, vol. 67, no. 4, 15 February 2007 (2007-02-15), pages 1769-1774, XP008110896, ISSN: 0008-5472, DOI: 10.1158/0008-5472.CAN-06-3783

## Description

### FIELD OF THE INVENTION

The present invention is directed to methods and compositions for treating cancer, including, specifically, hematologic malignancies, and including, more specifically, B-cell malignancies, with anti-tumor antibody-tumor suppressor fusion proteins in order to selectively restore tumor suppressor gene function to cancer cells in which such tumor suppressor gene function has been lost. The present invention is also directed to methods and compositions for diagnosing cancer and for predicting and assessing response to treatment.

### BACKGROUND OF THE INVENTION

Conventional approaches to treating malignancies and to predicting and assessing their responses to specific treatment regimens rely on properly classifying the type of tumor present. Proper classification, in turn, relies primarily on clinical features, tumor cell morphology, tumor cell immunophenotype and, to a lesser extent, tumor cell chromosomal abnormalities. However, even within a given tumor type, response to specific treatment regimens is, often, quite variable, and analyses at the molecular level reveal that the tumor types defined by conventional classification schemes are, often, quite heterogeneous.

Recent efforts to classify tumors, including hematologic malignancies, have, therefore, focused on identifying the specific genetic abnormalities that drive the growth of specific tumor types. Such genetic abnormalities can then serve as markers of disease and/or as targets for therapy.

Follicular lymphomas (FLs) are among the most common B-cell malignancies. FLs are characterized by a t(14;18)(q32;q21) chromosomal translocation that results in constitutive expression of the anti-apoptotic B-cell CLL/lymphoma 2 (Bcl2) protein. However, lymphomagenesis and disease progression require additional genetic lesions (Bende, Smit and van Noesel 2007). Amplification of *c-MYC,* loss of *p53* and deletions of chromosome 6q have all been associated with progression of B-cell lymphomas and shortened survival (Johnson, et al. 2009) (Nanjangud, et al. 2007). The exact nature of these molecular events is only incompletely understood. Clinical outcome for patients with B-cell lymphomas has improved with the addition of anti-CD20 antibody (e.g., rituximab) to conventional chemotherapeutic regimens. However, transplantation remains the only curative option for FL (Relander, et al. 2010).

Recent technological advances have facilitated the genome-wide detection of genetic and epigenetic changes in cancer. In parallel, RNA-interference (RNAi) technology and its adaptation to genetic screens have enabled the execution of rapid and unbiased loss-of-function studies in mammalian cells and *in vivo* (McCaffrey, et al. 2002). Together, these technologies can help uncover tumor suppressor genes that might not have been identified by genomic data analyses alone (Oricchio, et al. 2010).

The protein products of tumor suppressor genes can directly or indirectly prevent cell division or lead to cell death. Functional loss of tumor suppressor genes and/or their protein products through gene deletion, inactivating mutation or epigenetic mechanisms can result in uncontrolled cell growth and the development of cancer. Many tumors are known to result primarily from the functional loss of a tumor suppressor. However, due to the difficulties inherent in targeting tumor suppressor function specifically to those cancer cells in which such tumor suppressor function has been lost, restoration of tumor suppressor function to tumor cells has not, heretofore, been viewed as a practicable approach to the treatment of cancer.

### SUMMARY OF THE INVENTION

The present invention provides a fusion protein comprising a recombinant immunoglobulin (Ig) heavy chain or portion thereof having an amino-terminal variable region (Fv) that specifically binds to a lymphoma cell surface marker antigen, wherein said Ig heavy chain or portion thereof is linked at its carboxy-terminus by a peptide bond to the amino-terminal amino acid of tumor suppressor protein EphA7^{ECD}. In an embodiment, the lymphoma cell surface marker antigen is CD20 antigen. In an embodiment, the tumor suppressor protein EphA7^{ECD} may comprise SEQ ID NO:02. The present invention also provides a recombinant expression vector comprising a nucleotide sequence encoding the fusion protein.

The present invention provides the fusion protein or the expression vector for use as a medicament. The present invention also provides the fusion protein or the expression vector for use in a method for reducing one or more symptoms of cancer in a subject, wherein said cancer is lymphoma. In an embodiment, the cancer may be B cell lymphoma, follicular lymphoma, non-Hodgkin's lymphoma, Burkitt's lymphoma or large B-cell lymphoma. In an embodiment, the cancer comprises cancer cells that express a tumor cell surface marker that can be bound by the amino-terminal variable region (Fv) of a recombinant immunoglobulin (Ig), and that comprise one or more of a deletion of the ephrin receptor A7 (EPHA7) gene, reduced expression of the EphA7 protein, or increased expression of EphA2 protein. In an embodiment, the cancer cells express CD20. In an embodiment, the cancer cells are B cells. In an embodiment, the subject is human.

The present invention also provides *in vitro* methods for identifying cancer cells responsive to the fusion protein or the expression vector described above. The method may comprise determining in said cancer cells the presence of a deletion of ephrin receptor A7 (EPHA7) gene, wherein detecting deletion of said EPHA7 gene identifies said cancer cells as responsive to the fusion protein or expression vector; or may comprise determining expression of EphA7 protein by said cancer cells, wherein detecting reduced expression of said EphA7 protein identifies said cancer cells as responsive to the fusion protein or expression vector; or the method may comprise determining expression of EphA2 protein by said cancer cells, wherein detecting increased expression of said EphA2 protein identifies said cancer cells as responsive to the fusion protein or expression vector.

Accordingly, this invention is drawn to compositions for treating lymphoma, including B-cell malignancies, and for predicting and assessing response to treatment. In some embodiments, deletion of the ephrin receptor A7 gene (*EPHA7*) or loss of EphA7 expression can be used to identify a subset of lymphomas that will respond to treatment with a secreted, truncated EphA7 isoform comprising the extracellular domains of EphA7 (EphA7^{ECD}; sometimes referred to as EphA7^{TR}) or analogues thereof. In other embodiments, administration of a pharmaceutical composition comprising an anti-CD20 antibody-EphA7^{ECD} fusion protein (anti-CD20-EphA7), in which EphA7^{ECD} is fused downstream of the rituximab (Rituxan®/MabThera®) immunoglobulin G1 (IgG1) constant region, can be used to treat such lymphomas. In some other embodiments, deletion of *EPHA7,* loss of EphA7 expression and/or increased cell surface expression of EphA receptors, including EphA2, can be used to identify tumors likely to respond to treatment with EphA7^{ECD} or analogues thereof. In yet other embodiments, administration of a pharmaceutical composition comprising an anti-tumor antibody-EphA7^{ECD} fusion protein can be used to treat such tumors.

The present application also discloses an anti-tumor antibody-tumor suppressor fusion protein comprising: (1) a recombinant immunoglobulin (Ig) heavy chain or portion thereof having an amino-terminal variable region (Fv) specific for a cell-surface antigen of a tumor cell; and (2) said heavy chain or portion thereof being joined at its carboxy-terminus by a peptide bond to the amino-terminal amino acid of a tumor suppressor protein or functional portion thereof. The Ig heavy chain of the anti-tumor antibody-tumor suppressor fusion protein may comprise the Fv of rituximab. The Ig heavy chain Fv of the anti-tumor antibody-tumor suppressor fusion protein may be specific for CD20. The Ig heavy chain Fv may be specific for a cell-surface antigen found on malignant B-cells. The Ig heavy chain Fv may be specific for a cell-surface antigen found on the cells of a hematologic tumor. The Ig heavy chain Fv may be specific for a cell-surface antigen of the cells of a solid tumor. The tumor suppressor protein may be EphA7^{ECD}. The tumor suppressor protein may be EphA7 or an EphA2-binding portion thereof.

The application also discloses a method for treating cancer comprising administering a therapeutically effective amount of any one or more of the anti-tumor antibody-tumor suppressor fusion proteins described herein.

The invention application additionally discloses a DNA construct or constructs encoding any one or more of the anti-tumor antibody-tumor suppressor fusion proteins described herein.

Also disclosed herein is a tumor suppressor immunoconjugate comprising: (1) a recombinant Ig heavy chain or portion thereof having an Fv specific for a cell-surface antigen of a tumor cell joined at its carboxy-terminus by a peptide bond to the amino-terminal amino acid of a tumor suppressor protein or functional portion thereof; and (2) an Ig light chain or portion thereof having an Fv specific for said cell-surface antigen, said Ig heavy and light chains or portions thereof forming together a functional antigen-binding site, such that said immunoconjugate displays both antigen-binding specificity and tumor suppressor activity.

The application further discloses a method for treating cancer comprising administering a therapeutically effective amount of any one or more of the tumor suppressor immunoconjugates described herein. The antigen-binding site may be the antigen-binding site of rituximab. The antigen-binding site may be specific for CD20. The antigen-binding site may be specific for a cell-surface antigen found on malignant B-cells. The antigen-binding site may be specific for a cell-surface antigen found on the cells of a hematologic tumor, or specific for a cell-surface antigen of the cells of a solid tumor. The tumor suppressor protein may be EphA7^{ECD}. The tumor suppressor protein may be EphA7 or an EphA2-binding portion thereof.

The application additionally discloses a DNA construct or constructs encoding any one or more of the tumor suppressor immunoconjugates described herein.
The application further discloses a method to identify tumors responsive to treatment with any one or more of the anti-tumor antibody-tumor suppressor fusion proteins described herein, by measuring the deletion or inactivation of the gene for said tumor suppressor and/or the loss of expression of said tumor suppressor protein. The tumor suppressor protein may be EphA7^{ECD}.

Also disclosed herein is a method to identify tumors responsive to treatment with any one or more of the tumor suppressor immunoconjugates described herein, by measuring the deletion or inactivation of the gene for said tumor suppressor and/or the loss of expression of said tumor suppressor protein. The tumor suppressor protein may be EphA7^{ECD}.

The application also discloses a method to identify tumors responsive to treatment with any one or more of the anti-tumor antibody-tumor suppressor fusion proteins described herein, by measuring cell-surface EphA receptor expression.

Also disclosed is a method to identify tumors responsive to treatment with any one or more of the tumor suppressor immunoconjugates described herein, by measuring cell-surface EphA receptor expression.

The application also discloses a fusion protein comprising a recombinant immunoglobulin (Ig) heavy chain or portion thereof having an amino-terminal variable region (Fv) that specifically binds to a tumor cell surface marker antigen, wherein the Ig heavy chain or portion thereof is linked at its carboxy-terminus by a peptide bond to the amino-terminal amino acid of tumor suppressor protein EphA7^{ECD}. The tumor suppressor protein EphA7^{ECD} may comprise SEQ ID NO: 02.

The application also discloses a recombinant expression vector comprising a nucleotide sequence encoding a fusion protein comprising a recombinant immunoglobulin (Ig) heavy chain or portion thereof having an amino-terminal variable region (Fv) that specifically binds to a tumor cell surface marker antigen, wherein the Ig heavy chain or portion thereof is linked at its carboxy-terminus by a peptide bond to the amino-terminal amino acid of tumor suppressor protein EphA7ECD.

The application additionally discloses a method for reducing one or more symptoms of cancer comprising administering to a subject in need thereof a therapeutically effective amount of at least one of (a) a fusion protein comprising a recombinant immunoglobulin (Ig) heavy chain or portion thereof having an amino-terminal variable region (Fv) that specifically binds to a tumor cell surface marker antigen, wherein the Ig heavy chain or portion thereof is linked at its carboxy-terminus by a peptide bond to the amino-terminal amino acid of tumor suppressor protein EphA7^{ECD}, and (b) a recombinant expression vector comprising a nucleotide sequence encoding a fusion protein comprising a recombinant immunoglobulin (Ig) heavy chain or portion thereof having an amino-terminal variable region (Fv) that specifically binds to a tumor cell surface marker antigen, wherein the Ig heavy chain or portion thereof is linked at its carboxy-terminus by a peptide bond to the amino-terminal amino acid of tumor suppressor protein EphA7ECD. The cancer may be lymphoma. The cancer may comprise cancer cells that a) express CD20 protein, and b) comprise one or more of i) deletion of ephrin receptor A7 (*EPHA7*) gene, ii) reduced expression of EphA7 protein, and iii) increased expression of EphA2 protein. The cancer cells may comprise B cells. The subject may be human.

### DETAILED DESCRIPTION OF THE INVENTION

### Introduction

FLs are among the most common types of non-Hodgkin's lymphoma (NHL). They are characterized by the translocation t(14;18)(q32;q21) and increased expression of *BCL2* (Bende, Smit and van Noesel 2007). Amplification of *c-MYC,* loss of *p53* and deletions of chromosome 6q have all been associated with progression and shortened survival in FL (Nanjangud, et al. 2007). Up to 20% of FLs sustain large and hemizygous deletions of chromosome 6q11-27, suggesting the presence of one or more tumor suppressor genes in this region (Offit, et al. 1993) (Gaidano, et al. 1992). Clinically, FL shows persistent growth and eventual progression. Outcomes have improved with the addition of the anti-CD20 antibody rituximab to standard chemotherapeutic regimens, but transplantation remains the only curative option for FL (Relander, et al. 2010).

We used an unbiased loss-of-function screen to complement genomic analyses of tumors. Using an RNAi library tailored to the 6q deletions seen in FL, we identified a secreted form of the EphA7 receptor (EphA7^{ECD}) (Holmberg, Clarke and Frisén 2000) as a tumor suppressor. Hemizygous loss of *EPHA7* occurs in 12% of FLs, and the gene is differentially silenced in up to 72% of FLs. *In vivo* knockdown of *EPHA7* accelerates lymphoma development in mouse models of FL. Conversely, the purified EphA7^{ECD} protein has striking anti-tumor effects on xenografted human lymphoma cells. Moreover, by fusing EphA7 to an anti-CD20 antibody, we are able to target EphA7's tumor suppressive activity to CD20⁺ lymphoma cells *in vivo.* Thus, we identify a surprising role for EphA7^{ECD} as an antitumor protein with significant therapeutic potential in lymphoma, and we describe a new strategy, use of anti-tumor antibody-tumor suppressor fusion proteins, to selectively restore tumor suppressor gene function to cancer cells in which such function has been lost.

### Genomic Analysis of 6q Deletions in FL and Burkitt's Lymphoma (BL)

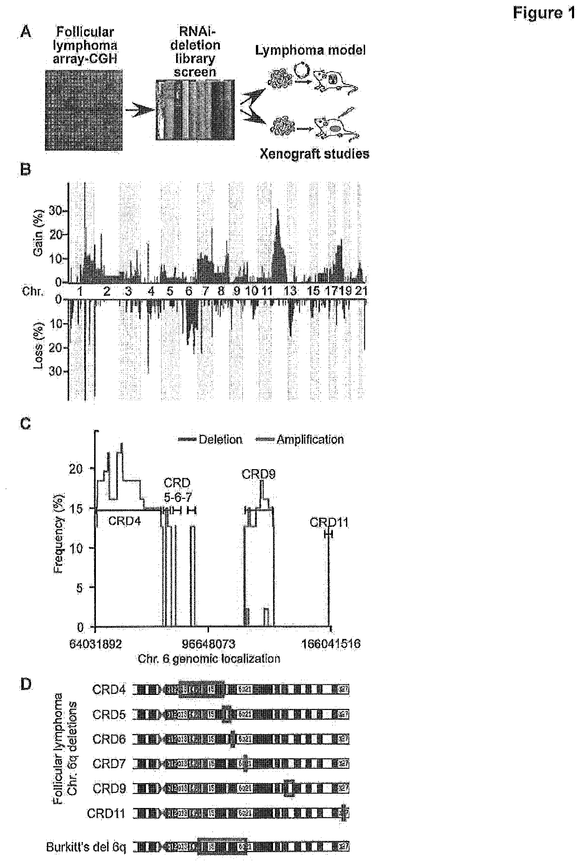

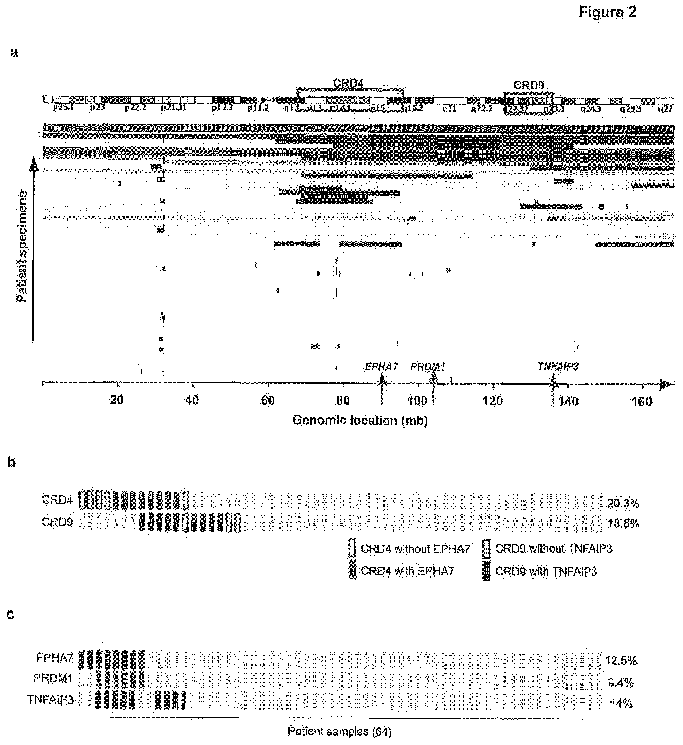

We conducted a systematic functional genomics study into the molecular pathogenesis of FL (Figure 1a). First, we analyzed 64 FLs representing pathological grades I-III (Grade I, 21; II, 23; IIIa, 16; IIIb, 8) by array-comparative genomic hybridization (aCGH). We observed 92 common (i.e., present in more than 10% of FLs tested) regions of deletion (CRD); 38 were tumor-specific and not found in the reference DNA, four were physiological and 50 were copy number variations (CNVs) (Figure 1b). Consistent with previous cytogenetic studies (Offit, et al. 1993) (Gaidano, et al. 1992) (Hauptschein, et al. 1998), we found that deletions affecting chromosome 6q11-27 occurred in 23% of FLs (15/64 cases). Individual cases showed a heterogeneous pattern of 6q loss (Figure 2).

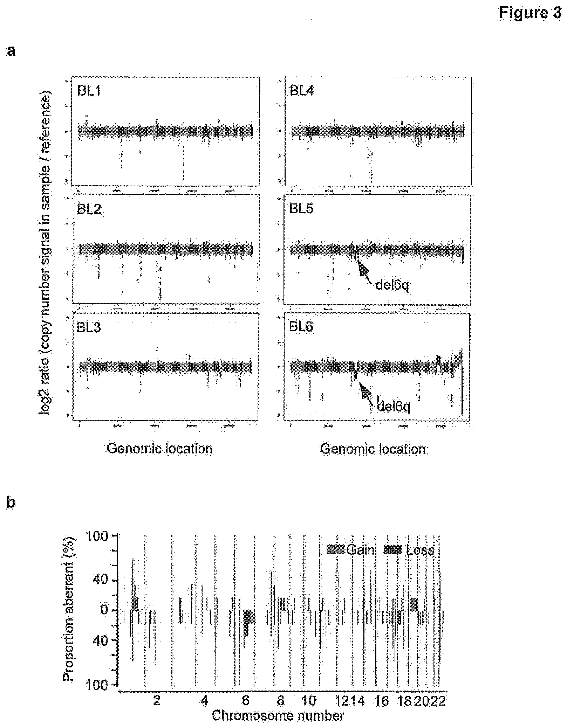

Cumulative analyses revealed CRDs that ranged from 5 kilobases (kb) (CRD11) to 27 megabases (Mb) (CRD4) and harbored between one and 78 genes (Figure 1c and 1d). Almost all the deletions were hemizygous, and small regions of apparent homozygous loss within CRD4 and CRD11 did not affect any genes. Analysis of six HIV-associated Burkitt's lymphomas (BLs) revealed partially overlapping 6q deletions in two cases (Figure 1d, Figure 3). Thus, while the size and complex patterns of hemizygous 6q deletions in FL suggest the presence of multiple tumor suppressor genes in this region, genomic data alone do not directly pinpoint a specific tumor suppressor gene.

### Unbiased RNAi Screen Identifies EPHA7 as a Tumor Suppressor Gene in 6q11-27

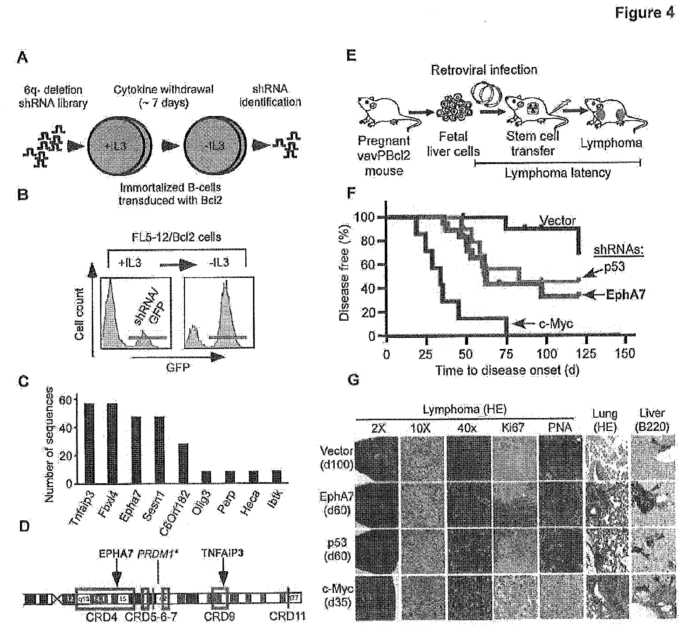

Given the complexity of 6q deletions in FL, we wondered whether an unbiased deletion-specific loss-of-function screen could point to potential tumor suppressor genes. We constructed a library of 260 short hairpin RNAs (shRNAs) targeting 84 genes (1 - 7 shRNAs per gene) in a murine stem cell virus (MSCV)-based, green fluorescent protein (GFP)-expressing vector. We used non-transformed murine pro-B lymphocytes (FL5-12 cells) engineered to express increased levels of Bcl2 as a surrogate *in vitro* system and screened for shRNAs that protect cells from cytokine (interleukin-3; IL-3) depletion (Mavrakis, et al. 2010) (Figure 4a). Briefly, we partially transduced FL5-12 cells overexpressing Bcl2 with the pooled 6q deletion library or empty vector and monitored for enrichment of cells expressing GFP (and shRNAs) following IL-3 depletion (Figure 4b). Sequencing identified the shRNAs present in the enriched population (Figure 4c), and individual re-testing confirmed a protective effect for shRNAs targeting the tumor necrosis factor, alpha-induced protein 3 (*TNFAIP3;* A20) and *EPHA7* genes (Figure 4d, Figures 5 and 6). Hence, our RNAi screen identifies a known tumor suppressor (*TNFAIP3*) (Compagno, et al. 2009) and points to *EPHA7* as a new candidate tumor suppressor gene.

### In Vivo Knockdown of EPHA7 Accelerates Lymphoma Development in Mouse Models of FL

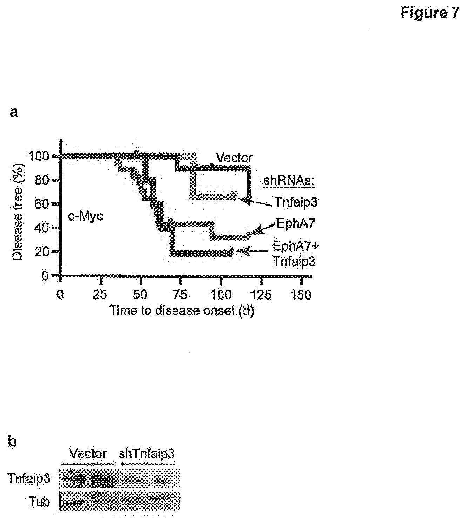

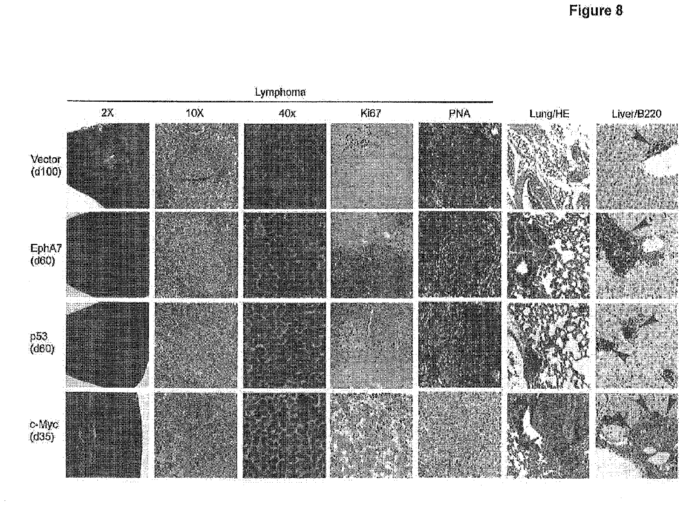

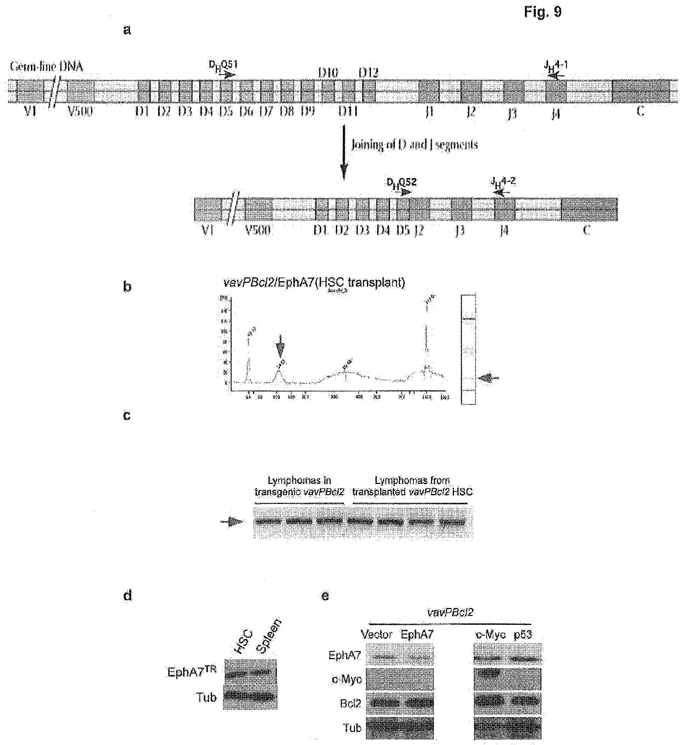

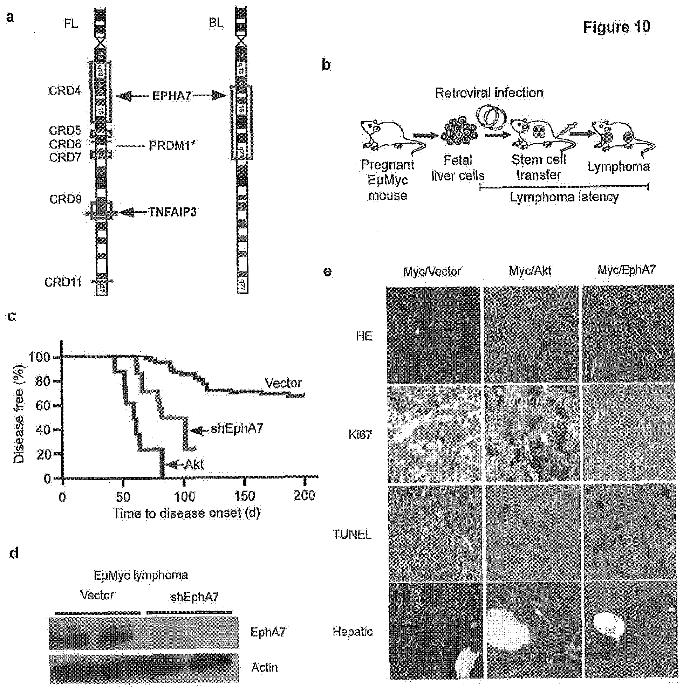

Next, we tested the effect of *EPHA7* in a mouse model of FL. Briefly, the vavP-BCL2 model recapitulates the genetics and morphology of human FL (Egle, et al. 2004). We transduced vavP-*BCL2* transgenic hematopoietic progenitor cells (HPCs) with retroviral shRNA constructs and transplanted these genetically engineered cells into irradiated recipients (Wendel, et al. 2004) (Figure 4e). Ninety percent of control animals remained tumor free for more than 100 days (vector; n = 11). *c-MYC* and *p53* have established roles in FL transformation (Nanjangud, et al. 2007), and enforced *c-MYC* expression (p <0.01; n = 7) and *p53* knockdown (median survival 60 days, p <0.01; n = 9) both accelerated lymphomagenesis *in vivo. EPHA7* knockdown had an effect on tumor latency similar to that of *p53* knockdown (p <0.01; n = 18) (Figure 4f). Knockdown of *TNFAIP3* alone or in combination with knockdown of *EPHA7* in the vavP-*BCL2* model showed modest effects on lymphoma latency [p_{(*TNFAIP3*vs. vector)} =0.28; n = 3 and p_{(*TNFAIP3*+*EPHA7*vs*. EPHA7*)} =0.93; n = 5)] (Figure 7). Analysis of tumors induced in vavP-BCL2 mice revealed features of FLs. These included follicular structures and peanut agglutinin (PNA) positivity, consistent with a germinal center (GC) B-cell phenotype. The lymphomas had overall low but heterogeneous Ki67, while apoptosis was absent. Only the vavP*-BCL2*/*c-MYC* tumors grew in a diffuse pattern resembling diffuse large B-cell lymphoma (DLBCL) (Figure 4g, Figure 8). All the tumors expressed B-cell markers (B220, CD19), and exhibited varying degrees of T-cell infiltration (Egle, et al. 2004). Sequencing of the immunoglobulin JH4 intron confirmed somatic hypermutation (Mandelbaum, et al. 2010) (Egle, et al. 2004) (McBride, et al. 2008). Polymerase chain reaction (PCR) analysis of the immunoglobulin heavy chain locus in tumors confirmed their clonal origin (Figure 9) (Egle, et al. 2004). Immunoblots revealed EphA7 expression in murine splenocytes and HPCs and partial knockdown in lymphomas (Figure 9d and 9e, Figure 10). Thus, *EPHA7* behaves as a tumor suppressor gene in a murine model that recapitulates many aspects of the genetics and pathology of FL.

We made similar observations regarding EphA7 in the Eµ-*MYC* model of pre-B-cell lymphoma (Adams, et al. 1985). Knockdown of *EPHA7* (n = 11) accelerated tumor development compared to vector (p <0.001; n = 60) (Figure 10).

### EPHA7 is Inactivated by Deletion and/or Promoter Methylation in Lymphoma

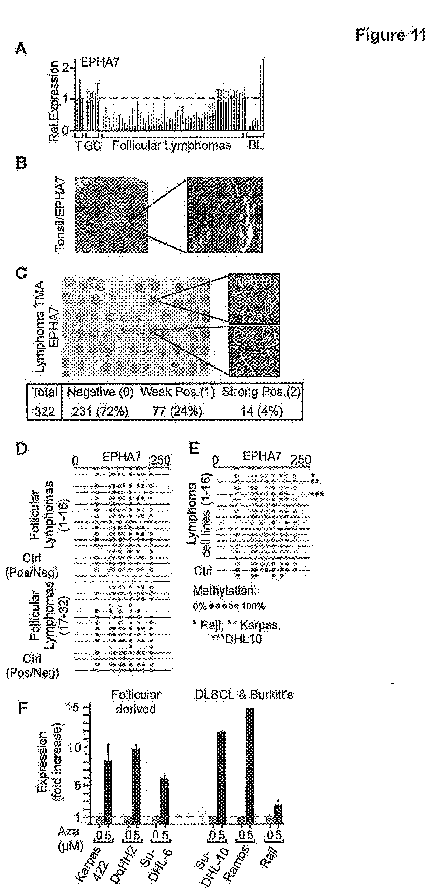

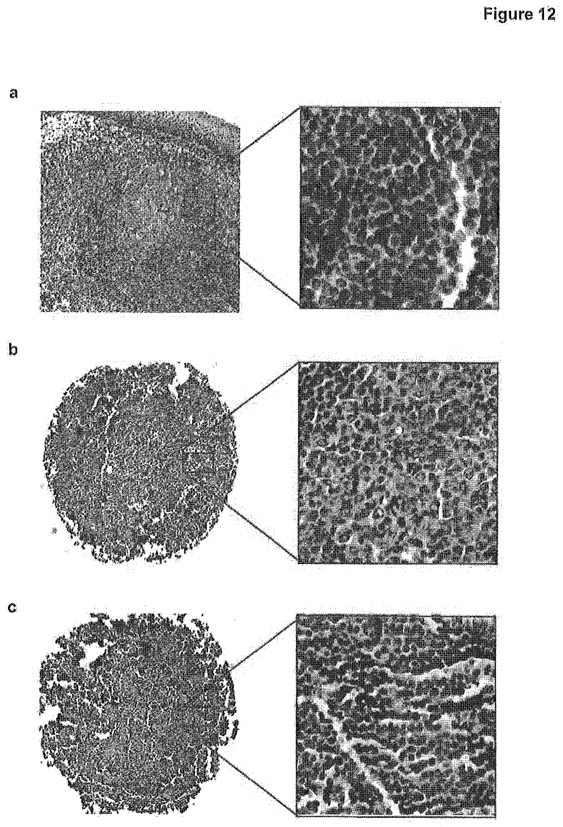

*EPHA7* is affected by hemizygous deletions in 12% of FL, and we wondered whether *EPHA7* might also be subject to epigenetic silencing or mutational inactivation. We noted a differential reduction of *EPHA7* expression levels in lymphoma cells compared to GC B-cells (Figure 11a and 11b). We did not detect *EPHA7* mutations in FL. However, quantitative reverse transcription-PCR (qRT-PCR) revealed decreased *EPHA7* mRNA levels in purified lymphoma cells. Specifically, 41 of 50 FLs (82%) and four of six BLs (67%) exhibited decreased *EPHA7* mRNA levels compared to B-cells from GC or tonsils (p_{(lymphoma vs. B-cell)} <0.02) (Figure 3a). Similarly, the EphA7 protein was easily detected in normal tonsils by immunohistochemistry but completely absent in 231 of 332 FL samples (72%) on a tissue microarray (TMA) (Figure 11b and 11c and Figure 12).

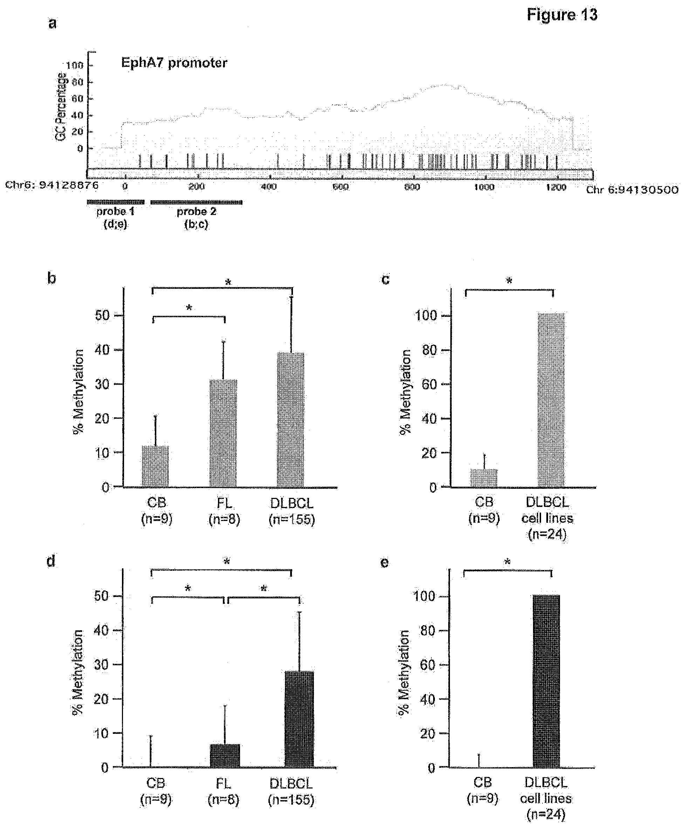

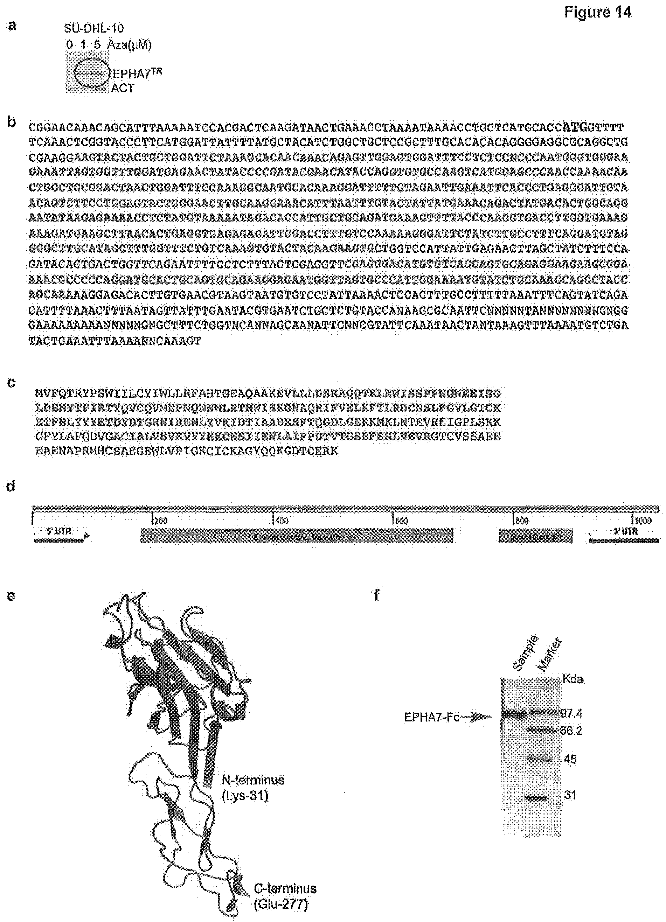

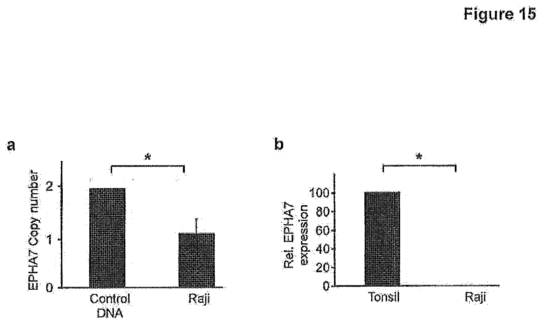

Mass spectrometric analysis (MassARRAY) of the *EPHA7* promoter in 32 primary FLs and 16 lymphoma cell lines revealed extensive CpG island methylation consistent with epigenetic gene silencing (Figure 11d and 11e). Analysis of a second panel of cells using the Hpall tiny fragment enrichment by ligation-mediated PCR (HELP) assay for methylation detection confirmed differential methylation of the *EPHA7* promoter in lymphoma cells (9 FLs, 155 DLBCLs and 24 lymphoma cell lines) vs. GC B-cells (n = 9), the normal counterpart of the lymphomas tested (Figure 13). Concordantly, *in vitro* treatment of human lymphoma cells with 5-aza-2'-deoxycytidine caused re-expression of *EPHA7* (Figure 11f, Figure 14). The effect was less pronounced in Raji cells, which have only one copy of the *EPHA7* gene (Figure 15). Thus, loss of *EPHA7* expression in lymphomas is due to differential methylation of the *EPHA7* promoter. Consistent with our observations, differential silencing of *EPHA7* has been reported in murine lymphomas and human B-lymphoblastic leukemias (B-ALLs) (Dawson, et al. 2007) (Kuang, et al. 2010). Hence, evidence of differential epigenetic silencing supports the role of *EPHA7* as a tumor suppressor gene in B-cell malignancies.

### B-cells Express Only A Secreted, Truncated Isoform Comprising the Extracelluar Domains of EphA7

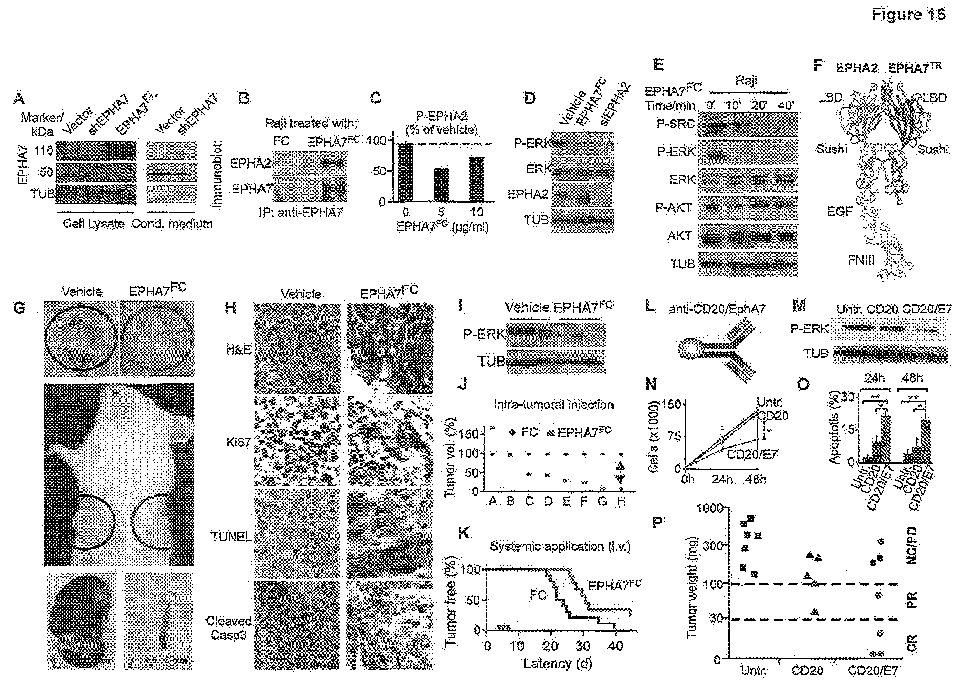

Ephrin receptors are tyrosine kinases that form dimers and are activated upon contact with ephrin-expressing cells (Seiradake, et al. 2010) (Himanen, et al. 2010). The role of ephrin signaling in cancer is unclear; both oncogenic and tumor suppressive functions have been proposed (Noren, et al. 2006) (Pasquale 2010). Alternate splicing of *EPHA7* produces a truncated protein (designated EphA7^{ECD} or EphA7^{TR}), which lacks the intracellular domains and the kinase activity of the full-length protein (Holmberg, Clarke and Frisén 2000) (Dawson, et al. 2007) (Valenzuela, et al. 1995). Murine B-lymphocytes and 5-aza-2'-deoxycytidine-treated SU-DHL-10 cells express only EphA7^{ECD} (Figure 16a, Figure 14), which is shed into the media (Figure 16a).

### EphA7^{ECD} Binds to EphA2, Blocking Activation of EphA2 and Src Kinases

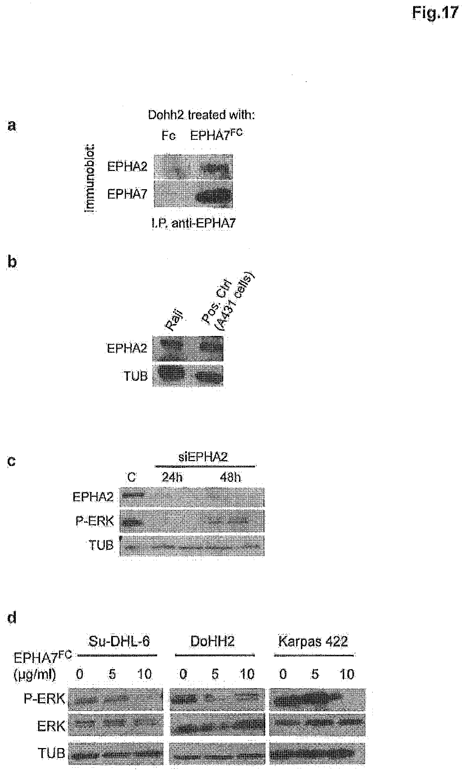

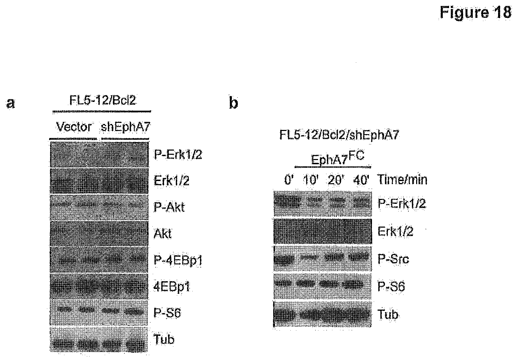

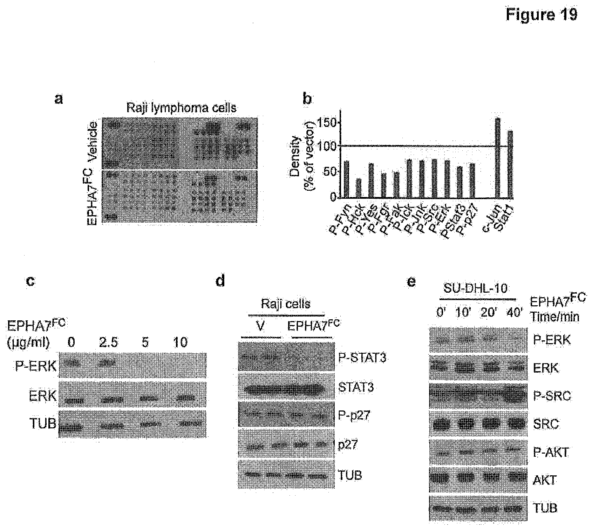

Immunoprecipitation of immunoglobulin Fc fragment-tagged EphA7^{ECD} (EphA7^{Fc}) demonstrates binding of EphA7 to the EphA2 receptor in Raji (Figure 16b) and FL-derived DoHH2 cells (Figure 17a). Enzyme-linked immunosorbent assay (ELISA) reveals inhibition of EphA2 phosphorylation by EphA7^{Fc} (Figure 16c). Both knockdown of EphA2 and administration of EphA7^{Fc} protein block Erk activation in Raji cells (Figure 16d and 16e) and in SU-DHL-6, DoHH2 and Karpas 422 cells (Figure 17c and 17d). Unlike tumor cells, the non-transformed FL5-12 lymphocytes express EphA7^{ECD}. Knockdown of *EPHA7* in FL5-12 cells activates Erk, and this activation is reversed upon treatment of the cells with EphA7^{Fc} (Figure 18). A phosphoprotein array identifies additional signaling effects of EphA7^{Fc} in Raji cells (Figure 19a and 19b). We confirmed effects on Erk, STAT3 and Src phosphorylation by immunoblot and note some differences between different lymphoma lines (Figure 16e, Figure 19d and 19e).

We modeled the interaction between EphA7 and EphA2 based on the known structure of EphA2 (Seiradake, et al. 2010) (Himanen, et al. 2010) and its homology with the EphA7 sequence (51%) and domain structure. Our model suggests an interaction through the receptors' Sushi and ligand binding domains (Figure 16f) suggesting that EphA7^{ECD} and EphA7^{Fc} function as decoy receptors, dimerizing with EphA2 on the cell surface and inhibiting EphA2 activation and signaling. More generally, our model suggests that EphA7^{ECD} and EphA7^{Fc} can dimerize with other EphA receptors on the cell surface and inhibit their activation and signaling as well.

### EphA7^{Fc} Exhibits Anti-Tumor Activity In Vitro and In Vivo

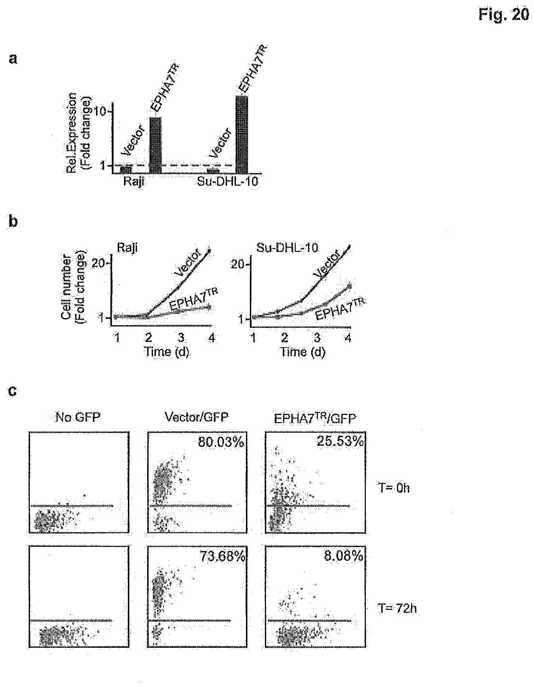

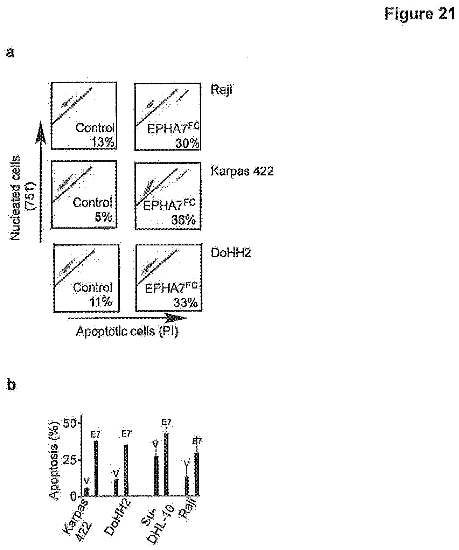

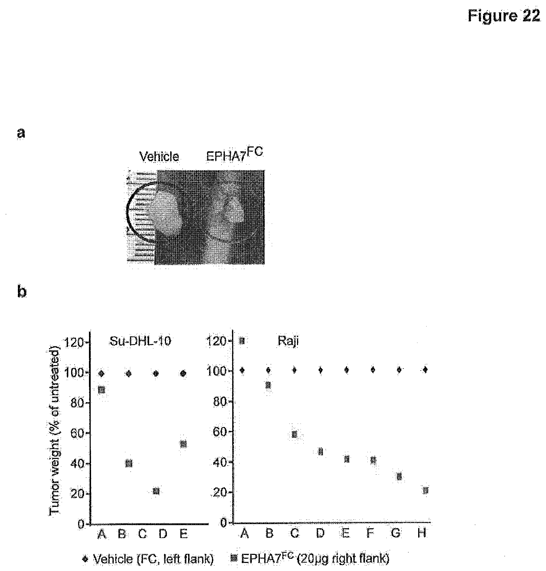

Restoration of *EPHA7* activity, by retroviral transduction or application of EphA7^{Fc}, has antiproliferative effects against Raji, SU-DHL-10, DoHH2 and Karpas 422 cells *in vitro* (Figures 20 and 21). Intravenous administration of purified EphA7^{Fc} (20µg/day for 3 days) resulted in dramatic regression of xenografted Raji and SU-DHL-10 tumors while vehicle (i.e., Fc)-treated tumors continued to grow (n = 12; p_{(EphA7Fc vs. Fc)} <0.04) (Figure 16g, Figure 22). Residual Eph7^{Fc}-treated Raji tumors exhibited extensive apoptosis, disrupted architecture and reduced Erk phosphorylation (Figure 16h - j). Systemic administration of EphA7^{Fc} in a prevention study (20µg/day intravenously for 3 days) was well tolerated and significantly delayed development of Raji lymphomas (EphA7^{Fc}, n = 5; Fc, n = 5; p <0.05) (Figure 16k). EphA7^{Fc} treatment of other tumor cell lines expressing high levels of cell-surface EphA2, including some breast cancer cell lines, resulted in significant inhibition of EphA2 signaling as assessed by Erk phosphorylation.

### Anti-Lymphoma Activity of Anti-CD20-EphA7^{ECD} Fusion Antibody Surpasses That of Anti-CD20 Antibody or EphA7^{ECD}

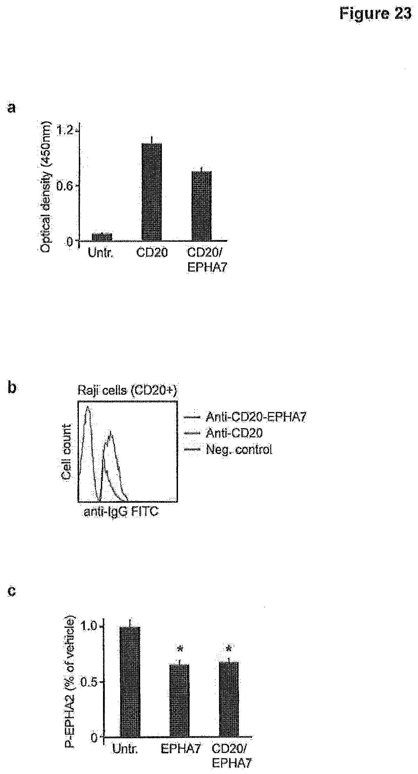

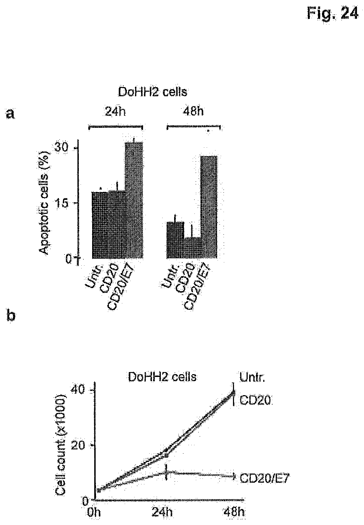

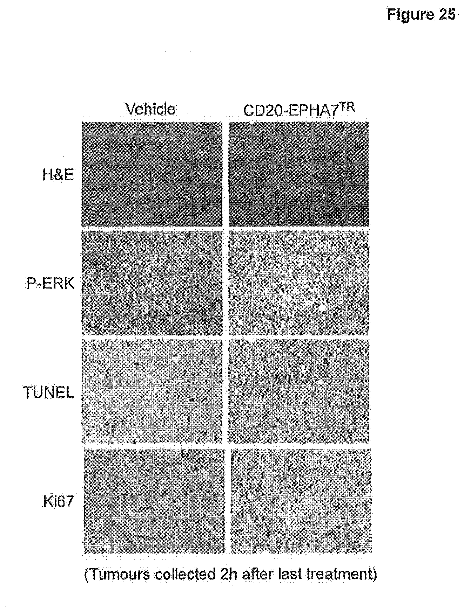

Next, we tested whether fusing EphA7^{ECD} to an anti-CD20 antibody (rituximab) could further enhance the therapeutic potential of EphA7^{ECD} (Figure 16l, Figure 23a). The fusion antibody (anti-CD20-EphA7) retains properties of both proteins. It recognizes CD20⁺ lymphoma cells and blocks EphA2 and Erk phosphorylation (Figure 16m, Figure 23b and 23c). The fusion antibody was also more efficient than anti-CD20 alone in slowing proliferation of, and killing, Raji or DoHH2 cells *in vitro* (Figure 16n and 16o, Figure 24). *In vivo* treatment with either anti-CD20 or anti-CD20-EphA7 (1µg/day intravenously for 5days) was well tolerated. In Raji xenografts (>1cm³ at time of treatment), administration of low-dose anti-CD20 antibody produced partial responses or slowed progression compared to vehicle (Fc). Only the fusion antibody produced complete responses (*ex vivo* tumor weight 0 - 30 mg) in 3 of 7 animals (p = 0.039, Fisher's exact for all three groups) (Figure 16p, Figure 25). Thus, anti-tumor antibodies can be used to selectively restore tumor suppressor function to cancer cells in which such function has been lost.

### MATERIALS AND METHODS

### Array-Comparative Genomic Hybridization (a-CGH)

DNA from fresh frozen or optimal cutting temperature compound-embedded tissue was isolated by the standard phenol-chloroform extraction method. DNA was quantified using a NanoDrop spectrophotometer (Thermo Fisher Scientific Inc.; www.nanodrop.com), its purity assessed by the ratio of absorptions at 260 nm vs. 280 nm and its integrity visualized on a 1% agarose gel with ethidium bromide. Prior to labeling each DNA sample and hybridizing it to an Agilent (Agilent Technologies; www.agilent.com) 244K oligonucleotide array, digestion efficiency was checked by incubating 1 µg of DNA with 1 µl of HaeIII restriction enzyme (10 U/µl) at 37°C for 2 hours and running the undigested and digested DNA (100 ng each) on a 1% agarose gel in parallel with a 1 kb DNA ladder. Human male DNA obtained from Promega Corporation (www.promega.com; Catalog# G147A) served as the reference DNA. Labeling and hybridization were performed according to protocols provided by Agilent. The slides were analyzed at 5 µm resolution using the Agilent G2565 Microarray Scanner System and Agilent Feature Extraction software (v9.1).

### DNA Copy Number Analysis

With the exception of Figure 16, which uses the UCSC March 2006 human reference sequence (hg18/NCBI Build 36), all genomic positions described in this study refer to UCSC May 2004 human reference sequence (hg17/NCBI Build 35) (http://genome.ucsc.edu/cgi-bin/hgGateway). The modified Circular Binary Segmentation (CBS) algorithm was used to identify segmental gains and losses along the autosomes. Change-points were defined as segments, corresponding to p-values <0.05. A CRD was defined as a gain and/or loss of two contiguous probes observed in >10% of the cases. A total of 92 CRDs were identified. Of these, four were physiological changes (B- and T-cell receptors), 50 were CNVs and the remaining 38 were tumor-specific. The Database of Genomic Variants (DGV), a catalogue of structural variations curated by The Centre for Applied Genomics (TCAG) (http://projects.tcag.ca/variation/) was used to identify and exclude CNVs. At the time of data analysis, the DGV comprised 8,083 entries that mapped to 3,933 genomic loci [variation.hg17.v2.txt; September 5, 2007 (Build 35/hg17)]. CGH data was further processed using Agilent's Feature Extraction software to quantitate the images. For normalization, we used a custom GC-normalization algorithm, which does a loess norm on both the total intensity of the probes and the local GC content in the genomic region surrounding the probes. The normalized data were segmented using DNAcopy, the standard CBS algorithm available from Bioconductor (http://www.bioconductor.org/help/bioc-views/release/bioc/html/DNAcopy.html). Each sample was then normalized to its own per-sample noise by dividing the segment means by the derivative noise (the mean absolute values of the difference between adjacent probes on the arrays). The next step used the RAE algorithm (Taylor, et al. 2008) to do a multi-sample analysis of the entire cohort. The RAE algorithm computes per-sample-calibrated thresholds, which can be used to compare signal levels across samples. The threshold function converts the log2 ratio signals from the CBS output via two logistic functions to a loss [-1,0] and gain [0-1] indicator output that is then averaged to give the genome-wide gain/loss recurrence frequency (plotted in Figure 1). For the chromosome 6q loss analysis, we used a global threshold of -0.5 for loss and 4.5 for homozygous deletion. Figure 3 shows the segment boundaries as computed by CBS with the vertical axis indicating the magnitude of the segment mean. See Taylor, et al. (2008) for additional details.

### Molecular Analysis of Murine Tumors

Genomic DNA was extracted from the lymphomas arising in transgenic vavP-*BCL2* mice and from the lymphomas derived from transplanted vavP-*BCL2* HSC. For DNA extraction, frozen tissue was submerged in liquid nitrogen then pulverized. The resulting powder was collected and transferred to a microfuge tube on ice. DNA was purified using the Gentra Puregene Cell Kit (Qiagen; www.qiagen.com) and diluted in water, and DNA quality was assessed by visualization after electrophoresis in a 1% agarose gel. DJ recombination in murine tumors was analyzed by nested PCR as described (Yu and Thomas-Tikhonenko 2002), and samples were analyzed using an Agilent 2100 Bioanalyzer and DNA 1000 Kit. For analysis of somatic hypermutation analyses, DNA samples were amplified as described (McBride, et al. 2008), and the PCR products were directly sequenced exactly as reported (Mandelbaum, et al. 2010).

### Quantitative DNA Methylation Analysis

Quantitative DNA methylation analysis was carried out using MassARRAY EpiTYPER from Sequenom, Inc. (www.sequenom.com). The MassARRAY EpiTYPER is a tool for the detection and quantitative analysis of DNA methylation using base-specific cleavage of bisulfite-treated DNA and Matrix-Assisted Laser Desorption/Ionization Time-of-Flight Mass Spectrometry (MALDI-TOF MS). Specific PCR primers for bisulfite-converted DNA were designed using Sequenom's EpiDesigner program (www.epidesigner.com). T7-promoter tags were added to the reverse primer to obtain a product that could be transcribed *in vitro,* and a 10-mer tag was added to the forward primer to balance the PCR conditions. Unmethylated cytosine in 1 µg of tumor DNA was converted into uracil by bisulfite treatment with the EZ-96 DNA Methylation Kit (Zymo Research Corporation; www.zymoresearch.com) according to the manufacturer's instructions. PCR reactions were carried out in duplicate with each of the two selected primer pairs, for a total of four replicates per sample. For each replicate, 1 ml of bisulfite-treated DNA was used as template for a 5-ml PCR reaction in a 384-well microtiter plate, using 0.2 units of KAPA2G Fast HotStart DNA Polymerase (Kapa Biosystems; www.kapabiosystems.com), 200 mM dNTPs and 400 nM of each primer. Cycling conditions were: 94°C for 15 minutes; 45 cycles of 94°C for 20 seconds, 56°C for 30 seconds, 72°C for 1 minute; and one final cycle at 72°C for 3 minutes. Unincorporated dNTPs were deactivated using 0.3 U of shrimp alkaline phosphatase (SAP) in 2 ml, at 37°C for 20 minutes, followed by heat inactivation at 85°C for 5 minutes. Two ml of SAP-treated reaction were transferred into a fresh 384-well microtiter plate, and *in vitro* transcription and T cleavage were carried out in a single 5-ml reaction mix, using the MassCLEAVE kit (Sequenom) containing 1x T7 polymerase buffer, 3 mM dithiothreitol (DTT), 0.24 ml of T Cleavage mix, 22 U T7 RNA and DNA polymerase and 0.09 mg/ml RNAse A. The reaction was incubated at 37°C for 3 hours. After the addition of a cation exchange resin to remove residual salt from the reactions, 10 nl of EpiTYPER reaction product was loaded onto a 384-element SpectroCHIP II array (Sequenom). SpectroCHIPs were analyzed using a Bruker Biflex III MALDI-TOF mass spectrometer (SpectroREADER, Sequenom). Results were analyzed using the EpiTYPER Analyzer software and manually inspected for spectra quality and peak quantification. *In vitro* treatment with 5-aza-2'-deoxycytidine was as described (Mavrakis, et al. 2008).

### HELP (HpaII tiny fragment enrichment by ligation-mediated PCR) Assay for DNA Methylation

DLBCL specimens were obtained from patients at the BC Cancer Agency in Vancouver or at Weill Cornell Medical Center. The use of human tissue was in agreement with research ethics boards of the Vancouver Cancer Center/University of British Columbia and Weill Cornell Medical Center. The HELP assay was performed as previously published (Shaknovich, Figueroa and Melnick 2010) using two probes located upstream of, and overlapping with, the transcriptional start site of the *EPHA7gene.* Products of HELP were hybridized to human HG17 custom promoter arrays (Roche NimbleGen, Inc.; www.nimblegen.com) covering 25,626 Hpall amplifiable fragments. Data quality control and analysis were performed as described (Thompson, et al. 2008). After quality control processing, a quintile normalization was performed on each array. DNA samples profiled by HELP were also subjected to bisulfite treatment and MassARRAY EpiTYPER analysis as previously described. In order to cover all possible sites of digestion, primers were designed to cover the outermost Hpall sites of the selected Hpall amplifiable fragments (HAF) as well as any other Hpall sites up to 2,000 base pairs upstream or downstream of the HAF. Correlation of MassARRAY results with normalized data from HELP assay revealed a Spearman's rank correlation of R=0.88. The adjusted linear regression model was used to obtain the conversion formula.

### Immunohistochemical and Tissue Microarray (TMA) Methods

The study cohort for analysis of EphA7 expression comprised FLs consecutively ascertained at the Memorial Sloan-Kettering Cancer Center (MSKCC) between 1985 and 2000. All cancer biopsies were evaluated at MSKCC, and the histological diagnosis was based on hematoxylin and eosin (H&E) staining. Use of tissue samples was approved by MSKCC's Institutional Review Board and Human Biospecimen Utilization Committee. TMAs were constructed as previously described (Scott, et al. 2007) except that a fully automated arrayer (Beecher Instruments ATA-27) was used. TMAs were pre-treated with Cell Conditioning Solution 1 (Ventana Medical Systems, Inc.; www.ventanamed.com), incubated with EphA7 rabbit polyclonal antibody from Abgent (www.abgent.com) at 1:50 dilution for 60 minutes and then stained with secondary anti-rabbit antibody from Vector Laboratories, Inc. (www.vectorlabs.com) at 1:200 dilution for 60 minutes. Cores were scored as 0, 1 or 2 where 0=no staining; 1= focal, weak staining; and 2 = moderate-to-strong staining in more than 50% of tumor cells.

### Antibody Production and Purification

To construct the anti-CD20-EphA7 antibody, we amplified the EphA7^{ECD} coding sequence by PCR from human genomic DNA. The PCR product was cloned into pAH6747, which contains an IgG1 constant region with an anti-CD20 heavy chain variable region (Dr. Sherie Morrison, UCLA). For antibody production, the anti-CD20-EphA7 heavy chain construct derived from pAH6747 and an anti-CD20 light chain (pAG10818) construct (Dr. Sherie Morrison, UCLA) were co-transfected into 293T cells, and the media in which the cells were growing was replaced every other day. Anti-CD20-EphA7 was purified from this conditioned media via affinity chromatography using recombinant Protein A. Briefly, the harvested media containing anti-CD20-EphA7 was dialyzed into 20 mM sodium phosphate (pH 7), passed over a 1 ml HiTrap rProtein A FF column (GE Healthcare Life Sciences; www.gelifesciences.com) and eluted with 100 mM glycine-HCl (pH 2.7). Eluant was collected in glass fraction tubes and immediately neutralized with 75 µl 1M Tris-HCl (pH 9.0) per ml of eluant. The antibody-containing peak fractions were pooled, dialyzed into phosphate-buffered saline and sterile filtered. Purity was determined by sodium dodecyl sulfate-polyacrylamide gel electrophoresis (SDS-PAGE), and antibody-fusion product concentration was determined with a spectrophotometer (280 nm) using an extinction coefficient of 1.43. ELISA using anti-human IgG (H+L) (Jackson ImmunoResearch Laboratories, Inc.; www.jacksonimmuno.com; #709-005-149) and horseradish peroxidase-conjugated-anti-human IgG Fc (Jackson ImmunoResearch Laboratories, Inc.; #709-035-098) antibodies was used to verify protein purity and integrity.

### Cell Culture, Cell Viability and Proliferation Assays, Vectors and Pooled shRNA Library Screen

FL5-12 murine lymphocytes were stably transduced with *BCL2* (FL5-12/*BCL2*); IL-3 depletion studies and viral transductions were as described (Mavrakis, et al. 2008). Cell viability was assessed using the Guava Viacount Assay (Millipore Corporation; www.millipore.com and LDS751 cell-permeant nucleic acid stain (Invitrogen; www.invitrogen.com) as previously described (Mavrakis, et al. 2008). The retroviral constructs utilized are based on MSCV and include *BCL2* (Wendel, et al. 2004) and individual or pooled shRNA constructs (Dickins, et al. 2005). Pooled shRNA screening technology has been described (Mavrakis, et al. 2010). Briefly, the shRNA library was constructed by pooling the individually cloned shRNAs. The screen design is depicted in Figure 4a. FL5-12/*BCL2* cells were transduced at low multiplicity of infection with the library pool containing 262 shRNAs and subjected to 7 days of IL-3 depletion. After recovery of viability, samples were collected for DNA isolation. Integrated shRNAs were amplified by PCR, subcloned into the pGEM-T Easy Vector (Promega) and identified by shRNA sequencing. Individual 'hits' from the screen were re-tested in the same *in vitro* assay and confirmed using multiple shRNAs against the same genes. The shRNAs against *PTEN* and *p53* have been previously reported (Mavrakis, et al. 2010) (Wendel, et al. 2006).

### Generation, Treatment and Analysis of Tumors in Mice

The vavP-*BCL2* model of FL (Egle, et al. 2004) and the adoptive transfer of retrovirally transduced HPCs (Wendel, et al. 2004) have been described. Data were analyzed in Kaplan-Meier format using the log-rank (Mantel-Cox) test for statistical significance. H&E staining, terminal deoxynucleotidyl transferase dUTP nick end labeling (TUNEL) and analyses for Ki67, cleaved caspase, B220 and other surface markers were as described by Mavrakis, et al. (2008). Tumor xenografts were established by subcutaneous injection of 1 x 10⁶ Raji or SU-DHL-10 human lymphoma cells mixed with Matrigel (BD Biosciences; www.bdbiosciences.com) into the flanks of NOD/SCID (NOD.CB17-Prkdc(scid)/J) mice. Once tumors exceeded 1 cm³ in size, mice were treated by three intratumoral injections of vehicle or 20 µg EphA7^{FC} (Recombinant Mouse EphA7 Fc Chimera; R&D Systems; www.rndsystems.com). Tumors were weighed and volumes were measured as described (Bergers, et al. 1999). Systemic administration of EphA7^{FC} and anti-CD20-EphA7 was by tail vein injection.

### Western Blot Analysis, ELISA, and Protein Arrays

Immunoblots were performed from whole cell lysates or supernatant as described (Wendel, et al. 2004). Briefly, 50 µg of protein per sample were resolved on SDS-PAGE gels, transferred to Immobilon-P Transfer Membranes (Millipore) and probed with antibodies against EphA7 (Santa Cruz Biotechnology, Inc.; www.scbt.com; #sc-917 diluted 1:200), EphA2 (Millipore, Inc. #05-480 diluted 1:1000), Bcl2 (Santa Cruz Biotechnology, Inc. #sc-509 diluted 1:500), c-Myc (Santa Cruz Biotechnology, Inc. #sc-40 diluted 1:200), phosphorylated eIF4E-BP1 (Cell Signaling Technology, Inc.; www.cellsignal.com ; #9451 diluted 1:1000), phosphorylated Erk1/2 (Cell Signaling Technology, Inc. #9101 diluted 1:800), Erk1/2 (Cell Signaling Technology, Inc. #9102 diluted:1000), phosphorylated Src (Cell Signaling Technology, Inc. #2101 diluted 1:1000), phosphorylated S6 ribosomal protein (Cell Signaling Technology, Inc. # 2215 diluted 1:1000), phosphorylated Akt (Cell Signaling Technology, Inc. #4058 diluted 1:1000) and tubulin (Sigma-Aldrich Co.; www.sigmaaldrich.com; #T5168 diluted 1:5000). Blots were developed chemiluminescently using the Amersham ECL Western Blotting System (GE Healthcare Life Sciences). ELISA for phosphorylated EphA2 in Raji cell lysates was performed utilizing the Human Phospho-EphA2 DuoSet IC (R&D Systems #DYC4056-2) according to the manufacturer's instructions. A human phosphoprotein detection array (R&D Systems #ARY003) was probed with cell lysates according to the manufacturer's instructions.

### Production of EphA7^{Fc} Protein

In addition to the EphA7^{Fc} protein obtained from commercial sources (R&D Systems; see above), we produced an identical protein using a baculoviral expression system. A DNA fragment corresponding to EphA7 amino acids Lys31 through Asn525 was cloned into the BamH1/Not1 sites of the pAcGP67B-based (BD Biosciences) pMA152, a baculovirus vector with an IgG Fc-tag at the C-terminus of the protein-coding region (Antipenko, et al. 2003) (Xu, et al. 2008). The recombinant baculovirus constructs were co-transfected, with BaculoGold linearized baculovirus DNA (BD Biosciences), into Sf9 insect cells. Passage four recombinant baculovirus was used to infect Hi-5 cells in suspension at a density of 1.8 x 10⁶ cells/ml in Sf-900 II SFM protein-free insect cell culture medium (Invitrogen). Infected cells were grown at 27°C and 100 rpm and harvested after 64 hours. Hi-5 cell supernatant containing the secreted EphA7^{Fc} was loaded onto a Protein A Sepharose column and eluted by a step-wise pH gradient fractionation in 100 mM glycine. The yield was 1-2 mg protein per liter of Hi-5 cell suspension (Antipenko, et al. 2003) (Xu, et al. 2008).

### qRT-PCR

Total RNA was extracted from tumor samples and cell lines using the AllPrep DNA/RNA/Protein Mini Kit (Qiagen). cDNA synthesis, real-time-PCR and analysis by the ΔΔ Ct method were performed as described (Mavrakis, et al. 2008). EphA7 PCR utilized TTTCAAACTCGGTACCCTTCA as forward primer and CATTGGGTGGAGAGGAAATC as reverse primer and SYBR green detection; glyceraldehyde 3-phosphate dehydrogenase (gapdh) was used as a standard (GAGTCAACGGATTTGGTCGT forward primer, GACAAGCTTCCCGTTCTCAG reverse primer and SYBR green detection); and human beta glucuronidase (gusb; Applied Biosystems; www.appliedbiosystems.com; #4333767F) was used as an endogenous control.

### PCR Amplification and Sequencing of Genomic DNA

Sequencing of genomic DNA was performed as described (Veeriah, et al. 2010). Genomic DNA was amplified using a REPLI-g Midi Kit (Qiagen). The exonic regions of interest (NCBI Build 36.1) were broken into amplicons of 500 bp or less, and the Primer3 program was used to design specific primers covering exonic regions plus at least 50 base pairs of flanking intronic sequence. M13 tails were added to facilitate Sanger sequencing. PCR reactions were carried out in 384-well plates in a Duncan DT-24 (KBiosystems Limited; www.kbiosystems.com) water bath thermal cycler, with 10 ng of amplified DNA as template, using a touchdown PCR protocol with Taq HotStart DNA Polymerase (Kapa Biosystems). The touchdown cycling conditions were: 95°C for 5 minutes; three cycles of 95°C for 30 seconds, 64°C for 30 seconds, 72°C for 60 seconds; three cycles of 95°C for 30 seconds, 62°C for 30 seconds, 72°C for 60 sec; three cycles of 95°C for 30 sec, 60°C for 30 seconds, 72°C for 60 seconds; 37 cycles of 95°C for 30 seconds, 58°C for 30 seconds, 72°C for 60 seconds; and one final cycle at 70°C for 5 minutes. The resulting DNA sequencing templates were purified using Agencourt AMPure (Beckman Coulter, Inc.; www.beckmangenomics.com). The purified PCR reaction products were split in two, and sequenced bidirectionally with M13 forward and reverse primers and BigDye Terminator v3.1 Cycle Sequencing kit (Applied Biosystems). Dye terminators were removed using the Agencourt CleanSEQ kit (Beckman Coulter, Inc.), and sequence reactions were run on an ABI PRISM 3730xl sequencing apparatus (Applied Biosystems).

### Mutation Detection

Mutations were detected using an automated detection pipeline at the MSKCC Bioinformatics Core. Bi-directional sequencing reads and mapping tables (to link read names to sample identifiers, gene names, read direction and amplicon) were subjected to a filter that excludes reads that have an average Phred score of <10 for bases 100-200. Passing reads were assembled against the reference sequences for each gene, containing all coding and untranslated exons including 5 kb upstream and downstream of the gene, using command line Consed 16.0 (Gordon, Abajian and Green 1998). Assemblies were passed on to PolyPhred 6.02b (Nickerson, Tobe and Taylor 1997), which generated a list of putative candidate mutations, and to PolyScan 3.0 (Chen, et al. 2007), which generated a second list of putative mutations. The lists were merged together into a combined report, and the putative mutation calls were normalized to '+' genomic coordinates and annotated using the Genomic Mutation Consequence Calculator. The resulting list of annotated putative mutations was loaded into a PostgreSQL database along with select assembly details for each mutation call (assembly position, coverage and methods supporting mutation call). To reduce the number of false positives generated by the mutation detection software packages, only point mutations supported by at least one bi-directional read pair and at least one sample mutation called by PolyPhred were considered, and only putative mutations that are annotated as having nonsynonymous coding effects, occur within an exon or within 11 base pairs of an exon boundary, or have a conservation score >0.699 were included in the final candidate list. Indels called by any method were manually reviewed and included in the candidate list if found to hit an exon. All putative mutations were confirmed by a second PCR and sequencing reaction, in parallel with amplification and sequencing of matched normal tissue DNA. All traces for mutation calls were manually reviewed.

### DESCRIPTION OF FIGURES

**Figure 1****. Oncogenomic study to identify tumor suppressor genes in FL. a.** The study design combines genomic tumor analyses with an RNAi screen and validation in murine models and in xenografts; **b.** aCGH analysis of 68 FLs showing frequencies of genomic gain (red) and loss (blue) across the genome; **c.** High resolution depiction of recurrent gains (red) and losses (blue) affecting chromosome 6q with CRDs indicated; **d.** Mapping of CRDs. The observed 6q deletions are typically large and hemizygous and do not readily identify a target gene.
**Figure 2****. Case-by-case analysis of gains and losses across chromosome 6q in FLs. a.** Regions of copy number loss and gain aligned with a map of chromosome 6. Losses are indicated in blue and gains in red with color intensity representing the signal strength. Location of some key genes as well as of CRD4 and CRD9 is indicated. **b.** *EPHA7* is affected in eight of 13 deletions involving CRD4; similarly *TNFAIP3* is affected in nine of twelve cases harboring CRD9 deletions. **c.** *EPHA7, PRDM1* and *TNFAIP3* are frequently co-deleted. Among 64 FL cases, deletions affect *EPHA7* in eight (12.5%), *PRDM1* in six (9.4%) and *TNFAIP3* in nine (14%). In five of 64 FLs (7.8%), all three genes are affected. The deletions are almost entirely hemizygous, and no gene is directly affected by bi-allelic loss. The size of the deletions and the complex pattern suggest multiple targets; however the genomic data alone do not pinpoint any specific gene associated with FL.
**Figure 3****. aCGH analysis of six HIV**-**associated BLs. a.** Segmental analyses corrected for CNVs reveals 6q deletions in two of six cases compared to a reference. **b.** Whole genome changes and the proportion of BLs affected. The smallest common region of deletion in 6q is extends from nucleotide 84,341,552 through nucleotide 117,084,517, which overlaps with CRDs 4-7 in FLs and includes *EPHA7* and *PRDM1.*
**Figure 4****. RNAi screen and *in vivo* validation of candidate tumor suppressor genes. a.** Design of a pooled, deletion-specific shRNA library screen in a surrogate model (immortalized FL5-*12*/*BCL2* cells). **b.** Fluorescence-activated cell sorting (FACS) analyses of cells transduced with the pooled 6q deletion library showing enrichment of cells expressing shRNAs (and the GFP reporter) following IL-3 depletion. **c.** Absolute number and identity of shRNA sequences retrieved from the enriched population. **d.** *EPHA7* and *TNFAIP3* map, respectively, to the CRD4 and CRD9 in FL; *PRDM1* did not score in this screen. **e.** Adoptive transfer enables genetic studies in the vavP-*BCL2* model of FL. **f.** Tumor latencies for animals receiving vavP-*BCL2* transgenic HPCs transduced with empty vector (black, n = 11), or shRNAs against *EPHA7* (red, n = 18) or *p53* (green, n = 9) or over-expressing *c-MYC* (blue, n = 7). **g.** Microscopic pathology and immunohistochemistry of vavP-*BCL2* lymphomas expressing the indicated constructs. Red arrows indicate infiltrating tumor cells.

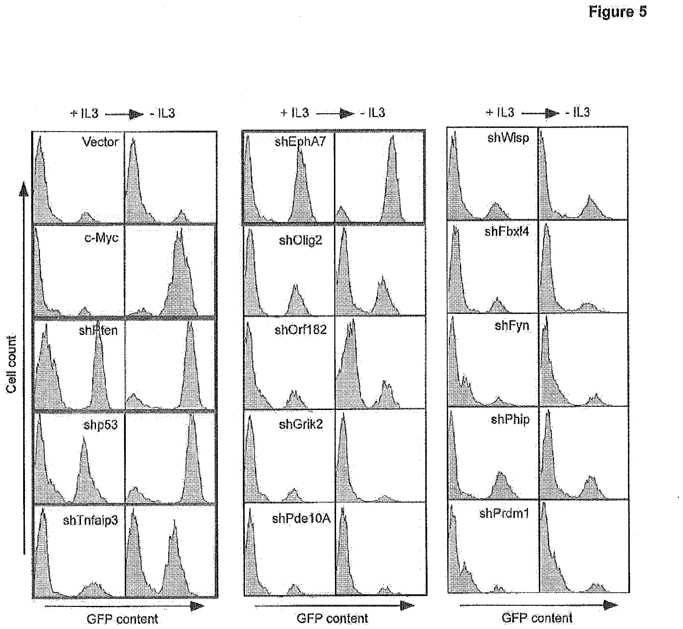

**Figure 5****. Individual validation of the screen results.** FL5-12/*BCL2* cells partially transduced with indicated shRNA/GFP or control constructs are shifted into IL-3-deficient media and monitored for changes in the proportion of GFP-expressing cells. An increase in the proportion of GFP-expressing cells (indicated with red outline) implicates co-transduced genes in the survival and proliferation of Bcl2-expressing B-cells upon IL-3 depletion.

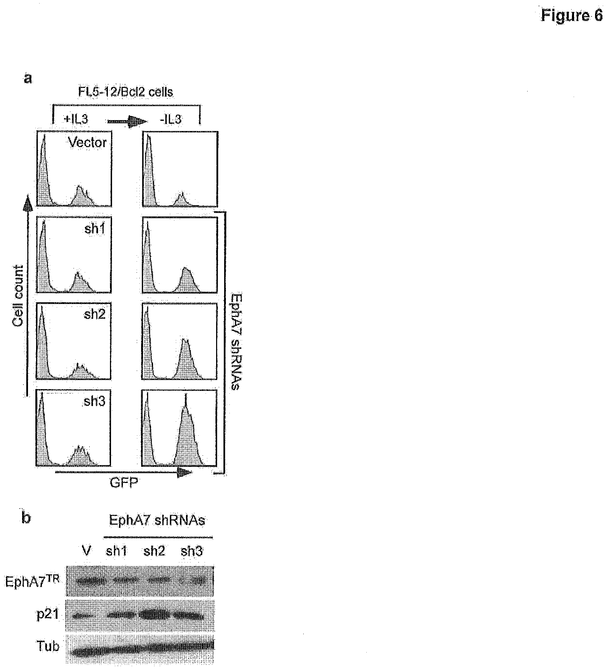

**Figure 6****. Characterization of *EPHA7* shRNAs**. **a.** Assessment of three shRNAs (sh1-3) targeting *EPHA7* in FL5-12/*BCL2* cells as described in Figure 5. **b.** Lysates of FACS-sorted FL5-12/*BCL2* cells expressing one of three shRNAs targeting *EPHA7* (sh1-3) or vector (V) blotted and probed with antibodies as indicated. (p21 has been reported as a common off-target of shRNAs.)
**Figure 7****. Role of *TNFAIP3* in vavP-*BCL2* HPC recipient animals. a.** Tumor latencies for animals receiving vavP-*BCL2* transgenic HPCs transduced with empty vector (black, n = 11), or shRNAs targeting *EPHA7* (red, n = 18), *TNFAIP3* (green, n = 3) or *EPHA7* and *TNFAIP3* (blue, n = 5). **b.** Lysates of FL5-12/*BCL2* cells expressing vector or an shRNA targeting TNFAIP3 blotted and probed with antibodies as indicated.
**Figure 8****. Microscopic pathology and immunohistochemistry of vavP-*BCL2* lymphomas.** Red arrows indicate infiltrating tumor cells.
**Figure 9****. Molecular characterization of murine vavP-*BCL2* lymphomas. a.** Location of primers used to analyze clonality. **b.** Nested PCR analysis of DJ recombination reveals a single band at ∼140bp, indicating clonality of this vavP-*BCL2*/*EPHA7* lymphoma. **c.** PCR analysis of DJ recombination in lymphomas arising in transgenic vavP-*BCL2* animals and in mice receiving HPCs transduced with the indicated shRNA constructs confirms their clonality. **d.** Lysates from vavP-*BCL2* transgenic HPCs (HSC) and splenocytes (spleen) blotted and probed with antibodies as indicated. **e.** Whole tumor lysates of vavP-*BCL2* tumors expressing *c-MYC* or shRNAs targeting *EPHA7, FOXO3* or *p53* and probed with antibodies as indicated.
**Figure 10****. Candidate tumor suppressor genes in the Eµ*-MYC* lymphoma model. a.** Diagram comparing the 6q deletions seen in FL and BL. **b.** the **Eµ*-MYC*** model and our HPC transplantation approach. **c.** Tumor latencies for animals receiving Eµ-*MYC* transgenic HPCs transduced with empty vector (black, n = 60, including concurrent and historic controls), with shRNA against *EPHA7* (red, n = 11) or with *AKT (blue,* n = 8); p_{(sh*EPHA7* or *AKT vs. vector*)} <0.05. **d.** Lysates from vector- or sh*EPHA7*-expressing Eµ*-MYC* lymphomas blotted and probed as indicated. **e.** Microscopic pathology and immunohistochemistry of **Eµ*-MYC*** lymphomas expressing the indicated constructs.
**Figure 11****. *EPHA7* is differentially silenced in FLs and expressed in GC B-cells. a.** qRT-PCR results for *EPHA7* in purified B-cells from reactive tonsils (T), GC, FLs and BLs. Results are displayed as mean ± standard deviation; p _{(tumor vs. normal)} <0.05 for FL and BL. **b.** Immunohistochemical detection of the EphA7 protein in a normal tonsil. **c.** Representative section of TMA representing 322 human FLs stained for EphA7. **d and e.** MassARRAY analysis of *EPHA7* promoter methylation in 32 FLs **(d)** and 16 human lymphoma lines **(e)** and positive/negative controls (Ctrl); the color scale indicates the degree of methylation with red indicating 0% and yellow 100%. **f. qRT-PCR** of *EPHA7* mRNA levels in human lymphoma cell lines treated with 5-aza-2'-deoxycytidine (Aza); p_{(Aza vs. untreated)} <0.01 for all cell lines; Raji cells are hemizygous for *EPHA7.*
**Figure 12****. Higher resolution EphA7 immunohistochemistry of human tissues. a.** EphA7 stain of normal tonsil. **b and c.** Representative negative **(b)** and positive **(c)** stains of human FLs on the TMA. (The TMA also includes, as controls, normal human kidney and liver and gastric cancers with known EphA7 staining patterns.)
**Figure 13****. HELP analysis of differential *EPHA7* promoter methylation in normal GC B-cells, FL, DLBCL and DLBCL-derived cell lines. a.** Location of the probes used to analyze *EPHA7* promoter methylation. **b and c.** Results with probe 1 comparing centroblasts (CB; n = 9) with FL (n = 8) and DLBCL (n = 155) **(b)** and CB with DLBCL-derived cell lines **(c). d and e.** Analogous results for probe 2. Silencing is progressive from GC B-cells to FL and aggressive DLBCL and nearly complete in DLBCL lines (* indicates p <0.05). The data are consistent with MassARRAY and expression data and indicate differential silencing between lymphomas and GC B-cells.
**Figure 14****. Characterization of EphA7^{ECD} expressed from a 5-aza-2'-deoxycytidine-treated human lymphoma line. a.** RT-PCR showing re-expression of the *EPHA7* transcript in SU-DHL-10 cells upon treatment with 1 µg and 5 µg 5-aza-2'-deoxycytidine (Aza). **b.** cDNA sequence (SEQ ID NO: 01) of the re-expressed RNA. **c.** Translation (SEQ ID NO: 02) of the cDNA sequence (SEQ ID NO: 01). **d.** Domain structure of the protein encoded by the re-expressed RNA. **e.** Predicted three-dimensional structure based on similarity with the known EphA2 structure. **f.** Gel purification of the Fc-tagged ectodomain of EphA7 (EphA7^{Fc}).
**Figure 15****. Analysis of the *EPHA7* locus and its expression in Raji lymphoma cells. a.** Genomic qPCR to determine *EPHA7* gene copy number in Raji lymphoma cells is consistent with loss of one allele of *EPHA7* in these cells. **b.** qRT-PCR of *EPHA7* mRNA confirms the absence of *EPHA7* expression in Raji cells compared to normal tonsils (* denotes p <0.05).
**Figure 16****. EphA7**^{Fc} **can block oncogenic signals and suppress the growth of xenografted human lymphomas. a.** Lysates and conditioned media from FL5-12/*BCL2* cells expressing empty vector, an shRNA targeting *EPHA7* (shEphA7) or full length *EPHA7* (EphA7FL) probed with an antibody against EphA7. **b.** Immunoprecipitation of lysates of Raji cells treated with EphA7^{Fc} (Fc-tagged ectodomain of EphA7) or with Fc control, immunoprecipitated with anti-EphA7 antibody and probed with antibodies to EphA7 or EphA2. **c.** ELISA for EphA2 phosphorylation in Raji cells treated with Eph7^{Fc} or Fc. **d.** Immunoblot of lysates of Raji cells treated with Fc control, EphA7^{Fc} (5 µg) or a small interfering RNA (siRNA) directed against EphA2. **e.** Immunoblot of lysates of Raji cells treated with 5µg/ml EphA7^{Fc} as indicated. **f.** Model of the EphA2 - EphA7^{ECD} interaction based on the known structure of EphA2 and its homology with EphA7 (LBD, ligand binding domain; EGF, EGF-like domain; FNIII, fibronectin domain). **g.** Xenografted Raji lymphomas grown in the flanks of NOD/SCID mice and treated three times on alternate days by intratumoral administration of 20 µg EphA7^{Fc} (red circle) or vehicle (Fc; black circle). **h.** Microscopic pathology of EphA7^{Fc}-treated and mock-treated Raji lymphomas stained as indicated. **i.** Immunoblot of lysates of tumors treated with EphA7^{Fc} or vehicle (Fc) *in vivo.* **j.** Matched-pair analysis of tumor volumes of eight (A-H) EphA7^{Fc}-treated (red) and vehicle (Fc)-treated (black) Raji lymphomas. **k.** Intravenous (i.v.) administration of EphA7^{Fc} (20µg/day x3 days; red) delays tumor development following injection of 1 x 10⁶ Raji lymphoma cells vs. administration of vehicle (Fc). **l.** Schematic of anti-CD20-EphA7 antibody. **m.** Immunoblot of lysates of Raji cells treated with anti-CD20 antibody (CD20), with anti-CD20-EphA7 antibody (CD20/E7) or untreated (Untr.). **n.** Proliferation of Raji cells treated as indicated; * denotes p_{(CD20/E7 vs. CD20)} <0.05. **o.** Apoptosis of Raji cells treated as indicated at 24 hours and 48 hours (* and ** denote p <0.05). **p.** Weight of tumors from mice bearing Raji xenografts (>1cm³) left untreated (Untr.) or given 1 µg/day x 5 days of anti-CD20 antibody (CD20) or anti-CD20-EphA7 antibody (CD20/E7). Tumors (in Matrigel) were collected two days after last treatment, weighed *ex vivo* and classified as complete response (CR; 0 - 30 mg), partial responses (PR; 30-100 mg) or no change/progressive disease (NC/PD; >100 mg).
**Figure 17****. EphA7 signaling effects in human lymphoma cell lines. a.** Immunoblot of lysates of DoHH2 cells treated with EphA7^{Fc} or Fc, immunoprecipitated with anti-EphA7 antibody and probed with antibodies to EphA7 or EphA2. **b.** Confirmation of EphA2 expression in Raji cells by immunoblot. **c.** Immunoblot of lysates of Raji cells treated with an siRNA against EphA2 for the indicated times and probed as indicated. **d.** Immunoblot of cell lysates of SU-DHL-6, DoHH2, and Karpas 422 cells treated with EPph7^{Fc} as indicated reveals some cell type-specific differences and similarities in signaling.
**Figure 18****. Signaling effects of EphA7^{Fc} and shRNA-mediated *EPHA7* knockdown. a.** FL5-*12*/*BCL2* cells expressing empty vector or shEphA7 probed with the indicated antibodies. **b.** FL5-12/*BCL2* cells expressing shEphA7 (FL5-12/*BCL2*/shEphA7) treated with EphA7^{Fc} (5 µg/ml) for the indicated times and probed as indicated.
**Figure 19****. EphA7^{Fc} affects several signaling molecules in human lymphoma cells. a.** Phosphoprotein array probed with lysates from Raji cells treated for 15 minutes with 5 µg/ml purified EphA7^{Fc} or vehicle. **b.** Quantification of phosphoprotein array results; the blue line indicates values for untreated cells. **c.** Dose-response relationship for EphA7^{Fc}-mediated Erk inhibition. **d and e.** Immunoblots of lysates of Raji (d) and SU-DHL-10 (e) lymphoma cells treated for 15 minutes with 5 µg/ml purified EphA7^{Fc} or vehicle (v) and probed for the indicated signaling molecules.
**Figure 20****. Retroviral expression of *EPHA7^{ECD}* in human lymphoma cells. a.** qRT-PCR showing the level of retroviral expression of *EPHA7^{ECD}* (EPHA7^{TR}) mRNA in Raji and SU-DHL-10 cells. **b.** Growth curve of FACS-sorted populations of vector- or *EPHA7^{ECD}* (EPHA7^{TR})-expressing Raji and SU-DHL-10 cells. **c.** Progressive depletion of *EPHA7^{ECD}*(EPHA7^{TR})/GFP-expressing Raji cells from mixed populations during 72 hours in *in vitro* culture.
**Figure 21****. Induction of apoptosis by EphA7**^{Fc} **in FL-derived cell lines *in vitro.* a.** FACS analysis of apoptosis induced by treatment with 5 µg EphA7Fc in Raji, Karpas 422 and DoHH2 cells. **b.** Quantification of apoptosis in a panel of lymphoma lines (V, Vehicle/Fc; E7, EphA7^{Fc}; p_{(E7 vs. V)} <0.05).
**Figure 22****. Matched-pair analysis of xenografted SU-DHL-10 and Raji human lymphoma cells treated with EphA7^{Fc} or Fc. a.** Photograph of tumors *ex situ* following treatment with 20µg EphA7^{Fc} or Fc (vehicle). **b.** Tumor weights comparing EphA7^{Fc}-treated and Fc-treated tumors (p <0.05 for SU-DHL-10 and Raji).
**Figure 23****. Purification and functional characterization of an anti-CD20-EphA7^{ECD} fusion antibody. a.** ELISA measurement of anti-CD20 and anti-CD20-EphA7antibody production. **b.** FACS analysis of CD20⁺ Raji cells treated with the anti-CD20 or anti-CD20-EphA7 antibodies and a fluorescein isothiocyanate (FITC)-labeled anti-IgG antibody. **c.** ELISA of EphA2 phosphorylation in Raji cells treated with Fc (Untr.), EphA7^{Fc} or anti-CD20-EphA7 (* indicates p_{(either treatment vs. vehicle)} <0.05).
**Figure 24****. Anti-CD20-EphA7 induces cell death and blocks proliferation of DoHH2 lymphoma cells. a.** Apoptosis of untreated (Untr.), anti-CD20 (CD20)- or anti- CD20-EphA7 (CD20/E7)-treated DoHH2 cells at 24 hours and 48 hours. **b.** Proliferation of DoHH2 cells untreated (Untr.), treated with anti-CD20 (CD20) or anti-CD2-EphA7 antibody (CD20/E7).
**Figure 25****. Microscopic histology and immunohistochemistry of Raji xenografts.** Tumors were treated with anti-CD20-EphA7 antibody (1µg/day x 5 days) or vehicle and collected two hours after the final treatment. The anti-CD20-EphA7-treated tumors reveal some increase in TUNEL positivity, reduced proliferation and reduced Erk phosphorylation.

### REFERENCES

Adams, J. M., et al. "The c-myc Oncogene Driven by Immunoglobulin Enhancers Induces Lymphoid Malignancy in Transgenic Mice." Nature 318, no. 6046 (December 1985): 533-538.
Antipenko, Alexander, et al. "Structure of the Semaphorin-3A Receptor Binding Module." Neuron 39, no. 4 (August 2003): 589-598.
Bende, R J, L A Smit, and C J van Noesel. "Molecular Pathways in Follicular Lymphoma." Leukemia 21, no. 1 (January 2007): 18-29.
Bergers, Gabriele, Kashi Javaherian, Kin-Ming Lo, Judah Folkman, and Douglas Hanahan. "Effects of Angiogenesis Inhibitors on Multistage Carcinogenesis in Mice." Science 284, no. 5415 (April 1999): 808-812.
Chen, Ken, et al. "PolyScan: An Automatic Indel and SNP Detection Approach to the Analysis of Human Resequencing Data." Genome Research 17, no. 5 (May 2007): 659-656.
Compagno, Mara, et al. "Mutations of Multiple Genes Cause Deregulation of NF-κB in Diffuse Large B-cell Lymphoma." Nature 459, no. 7247 (June 2009): 717-721.
Dawson, D. W., et al. "Global DNA Methylation Profiling Reveals Silencing of a Secreted Form of Epha7 in mouse and human germinal center B-cell lymphomas." Oncogene 26, no. 29 (June 2007): 4243-4252.
Egle, Alexander, Alan W. Harris, Mary L. Bath, Lorraine O'Reilly, and Suzanne Cory. "VavP-Bcl2 Transgenic Mice Develop Follicular Lymphoma Preceded By Germinal Center Hyperplasia." Blood 103, no. 6 (March 2004): 2276-2283.
Gaidano, Gianluca, et al. "Deletions Involving Two Distinct Regions of 6q in B-Cell Non-Hodgkin Lymphoma." Blood 80, no. 7 (October 1992): 1781-1787.
Gordon, David, Chris Abajian, and Phil Green. "Consed: A Graphical Tool for Sequence Finishing." Genome Research 8, no. 3 (March 1998): 195-202.
Hauptschein, Robert S., et al. "Cloning and Mapping of Human Chromosome 6q26-q27 Deleted in B-Cell Non-Hodgkin Lymphoma and Multiple Tumor Types." Genomics 50, no. 2 (June 1998): 170-186.
Himanen, Juha P., et al. "Architecture of Eph Receptor Clusters." Proceedings of the National Academy of Sciences of the United State of America 107, no. 24 (June 2010): 10860-10865.
Holmberg, Johan, Diana L. Clarke, and Jonas Frisén. "Regulation of Repulsion versus Adhesion by Different Splice Forms of an Eph Receptor." Nature 408, no. 6809 (November 2000): 203-206.
Johnson, Nathalie A., et al. "Lymphomas with Concurrent BCL2 and MYC Translocations: The Critical Factors Associated with Survival." Blood 114, no. 11 (September 2009): 2273-2279.
Kuang, Shao-Qing, et al. "Aberrant DNA Methylation and Epigenetic Inactivation of Eph Receptor Tyrosine Kinases and Ephrin Ligands in Acute Lymphoblastic Leukemia." Blood 115, no. 12 (March 2010): 2412-2419.
Mandelbaum, Jonathan, et al. "BLIMP1 is a Tumor Suppressor Gene Frequently Disrupted in Activated B Cell-like Diffuse Large B Cell Lymphoma." Cancer Cell 18, no. 6 (December 2010): 568-579.
Mavrakis, K. J., et al. "Tumorigenic Activity and Therapeutic Inhibition of Rheb GTPase." Genes & Development 22, no. 16 (August 2008): 2178-2188.
Mavrakis, Konstantinos J, et al. "Genome-wide RNA-mediated Interference Screen Identifies miR-19 Targets in Notch-induced T-cell Acute Lymphoblastic Leukaemia." Nature Cell Biology 12, no. 4 (April 2010): 372-379.
McBride, Kevin M., Anna Gazumyan, Eileen M. Woo, Tanja A. Schwickert, Brian T. Chait, and Michel C. Nussenzweig. "Regulation of Class Switch Recombination and Somatic Mutation by AID Phosphorylation." The Journal of Experimental Medicine 205, no. 11 (October 2008): 2585-2594.
McCaffrey, Anton P., Leonard Meuse, Thu-Thao T. Pham, Douglas S. Conklin, Gregory J. Hannon, and Mark A. Kay. "Gene Expression: RNA Interference in Adult Mice." Nature 418, no. 6893 (July 2002): 38-39.
Nanjangud, G., et al. "Molecular Cytogenetic Analysis of Follicular Lymphoma (FL) Provides Detailed Characterization of Chromosomal Instability Associated with the t(14;18)(q32;q21) Positive and Negative Subsets and Histologic Progression." Cytogenetics and Genome Research 118, no. 2-4 (November 2007): 337-344.
Nickerson, Deborah A., Vincent O. Tobe, and Scott L. Taylor. "PolyPhred: Automating the Detection and Genotyping of Single Nucleotide Substitutions Using Fluorescence-based Resequencing." Nucleic Acids Research 25, no. 14 (July 1997): 2745-2751.
Noren, Nicole K., Gabriele Foos, Craig A. Hauser, and Elena B. Pasquale. "The EphB4 Receptor Suppresses Breast Cancer Cell Tumorigenicity Through an Abl-Crk Pathway." Nature Cell Biology 8, no. 8 (August 2006): 815-825.
Offit, K., et al. "6q Deletions Define Distinct Clinico-Pathologic Subsets of Non-Hodgkin's Lymphoma." Blood 82, no. 7 (October 1993): 2157-2162.
Oricchio, Elisa, Andrew L. Wolfe, Jonathan H. Schatz, Konstantinos J. Mavrakis, and Hans-Guido Wendel. "Mouse Models of Cancer as Biological Filters for Complex Genomic Data." Disease Models & Mechansims 3, no. 11-12 (November - December 2010): 701-704.
Pasquale, Elena B. "Eph Receptors and Ephrins in Cancer: Bidirectional Signalling and Beyond." Nature Reviews Cancer 10, no. 3 (March 2010): 165-180.
Relander, Thomas, Nathalie A. Johnson, Pedro Farinha, Joseph M. Connors, Laurie H. Sehn, and Randy D. Gascoyne. "Prognostic Factors in Follicular Lymphoma." Journal of Clinical Oncology 28, no. 17 (June 2010): 2902-2913.
Scott, Clare L., et al. "Role of the Chromobox Protein CBX7 in Lymphomagenesis." Proceedings of the National Academy of Sciences of the United States of America 104, no. 13 (March 2007): 5389-5394.
Seiradake, Elena, Karl Harlos, Geoff Sutton, A. Radu Aricescu, and E. Yvonne Jones. "An Extracellular Steric Seeding Mechanism for Eph-Ephrin Signaling Platform Assembly." Nature Structural & Molecular Biology 17, no. 4 (April 2010): 398-402.
Shaknovich, Rita, Maria E. Figueroa, and Ari Melnick. "HELP (Hpall Tiny Fragment Enrichment by Ligation-Mediated PCR) Assay for DNA Methylation Profiling of Primary Normal and Malignant B Lymphocytes." Methods in Molecular Biology 632 (2010): 191-201.
Taylor, Barry S., et al. "Functional Copy-Number Alterations in Cancer." PLoS One 3, no. 9 (September 2008): e3179.
Thompson, Reid F., et al. "An Analytical Pipeline for Genomic Representations Used for Cytosine Methylation Studies." Bioinformatics 24, no. 9 (May 2008): 1161-1167.
Valenzuela, D. M., et al. "Identification of Full-length and Truncated Forms of Ehk-3, a Novel Member of the Eph Receptor Tyrosine Kinase Family." Oncogene 10, no. 8 (April 1995): 1573-1580.
Veeriah, Selvaraju, et al. "Somatic Mutations of the Parkinson's Disease-associated Gene PARK2 in Glioblastoma and Other Human Malignancies." Nature Genetics 42, no. 1 (January 2010): 77-82.
Wendel, H. G., et al. "Survival Signalling by Akt and elF4E in Oncogenesis and Cancer Therapy." Nature 428, no. 6980 (March 2004): 332-337.
Wendel, Hans-Guido, et al. "Loss of p53 Impedes the Antileukemic Response to BCR-ABL Inhibition." Proceedings of the National Academy of Sciences of the United States of America 103, no. 19 (May 2006): 7444-7449.
Xu, Kai, Kanagalaghatta R. Rajashankar, Yee-Peng Chan, Juha P. Himanen, Christopher C. Broder, and Dimitar B. Nikolov. "Host Cell Recognition by the Henipaviruses: Crystal Structures of the Nipah G Attachment Glycoprotein and its Complex with Ephrin-B3." Proceedings of the National Academy of Sciences of the United States of America 105, no. 29 (July 2008): 9953-9958.
Yu, Duonan, and Andrei Thomas-Tikhonenko. "A Non-transgenic Mouse Model for B-cell Lymphoma: In vivo Infection of p53-null Bone Marrow Progenitors by a Myc Retrovirus Is Sufficient for Tumorigenesis." Oncogene 21, no. 12 (March 2002): 1922-1927.

## Claims

1. A fusion protein comprising a recombinant immunoglobulin (Ig) heavy chain or portion thereof having an amino-terminal variable region (Fv) that specifically binds to a lymphoma cell surface marker antigen, wherein said Ig heavy chain or portion thereof is linked at its carboxy-terminus by a peptide bond to the amino-terminal amino acid of tumor suppressor protein EphA7^{ECD}.

2. A fusion protein according to claim 1 wherein the lymphoma cell surface marker antigen is CD20 antigen.

3. The fusion protein of claims 1 or 2, wherein said tumor suppressor protein EphA7^{ECD} comprises SEQ ID NO:02.

4. A recombinant expression vector comprising a nucleotide sequence encoding the protein of claims 1 or 2.

5. The fusion protein of claims 1-3 or the expression vector of claim 4 for use in a method for reducing one or more symptoms of cancer in a subject, wherein said cancer is lymphoma.

6. The fusion protein or expression vector for use according to claim 5 wherein said cancer is B cell lymphoma, follicular lymphoma, non-Hodgkin's lymphoma, Burkitt's lymphoma or large B-cell lymphoma.

7. The fusion protein or expression vector for use according to claim 5 wherein the said cancer comprises cancer cells that
a. express a tumor cell surface marker that can be bound by the amino-terminal variable region (Fv) of a recombinant immunoglobulin (Ig), and
b. comprise one or more of
i) deletion of ephrin receptor A7 (EPHA7) gene;
ii) reduced expression of EphA7 protein, and
iii) increased expression of EphA2 protein.

8. The fusion protein or expression vector for use according to claim 7 wherein said cancer cells express CD20.

9. The fusion protein or expression vector for use according to claim 7 or 8 wherein said cancer cells are B cells.

10. The fusion protein or expression vector for use according to claim 5 wherein said subject is human.

11. An *in vitro* method for identifying cancer cells responsive to the fusion protein of claims 1-3 or the expression vector of claim 4, comprising determining in said cancer cells the presence of a deletion of ephrin receptor A7 (EPHA7) gene, wherein detecting deletion of said EPHA7 gene identifies said cancer cells as responsive to the fusion protein or expression vector.

12. An *in vitro* method for identifying cancer cells responsive to the fusion protein of claims 1-3 or the expression vector of claim 4, comprising determining expression of EphA7 protein by said cancer cells, wherein detecting reduced expression of said EphA7 protein identifies said cancer cells as responsive to the fusion protein or expression vector of claim 4.

13. An *in vitro* method for identifying cancer cells responsive to the fusion protein of claims 1-3 or the expression vector of claim 4, comprising determining expression of EphA2 protein by said cancer cells, wherein detecting increased expression of said EphA2 protein identifies said cancer cells as responsive to the fusion protein or expression vector.

14. The fusion protein of claims 1-3 or the expression vector of claim 4 for use as a medicament.

## Patentansprüche

1. Ein Fusionsprotein, das eine schwere Kette eines rekombinanten Immunoglobulins (Ig) oder eines Teil davon mit einer amino-terminalen variablen Region (Fv) aufweist, die spezifisch ein Lymphomzell-Oberflächenmarker-Antigen bindet, wobei die besagte schwere Kette des Ig oder der Teil davon an ihrem Carboxy-Terminus über eine Peptidbindung mit der amino-terminalen Aminosäure des Tumorsuppressor-Proteins EphA7^{ECD} verbunden ist.

2. Ein Fusionsprotein nach Anspruch 1, wobei das Lymphomzell-Oberflächenmarker-Antigen ein CD20-Antigen ist.

3. Das Fusionsprotein nach den Ansprüchen 1 oder 2, wobei das besagte Tumorsuppressor-Protein EphA7^{ECD} SEQ ID NO:02 aufweist.

4. Ein rekombinanter Expressionsvektor, der eine Nukleotidsequenz aufweist, die das Protein nach Anspruch 1 oder 2 codiert.

5. Das Fusionsprotein nach den Ansprüchen 1-3 oder der Expressionsvektor nach Anspruch 4 für die Verwendung in einem Verfahren zur Reduzierung eines oder mehrerer Krebssymptome in einem Subjekt, wobei der besagte Krebs ein Lymphom ist.

6. Das Fusionsprotein oder der Expressionsvektor zur Verwendung nach Anspruch 5, wobei der besagte Krebs ein B-Zell-Lymphom, Follikuläres Lymphom, Non-Hodgkin-Lymphom, Burkitt-Lymphom oder ein großes B-Zell-Lymphom ist.

7. Das Fusionsprotein oder der Expressionsvektor zur Verwendung nach Anspruch 5, wobei der besagte Krebs Krebszellen aufweist, die
a. einen Tumorzell-Oberflächenmarker exprimieren, der durch die amino-terminale variable Region (Fv) eines rekombinanten Immunoglobulins (Ig) gebunden sein kann, und
b. eines oder mehrere aus
i) einer Deletion des Ephrinrezeptor-A7 (EPHA7)-Gens;
ii) einer reduzierten Expression des EphA7-Proteins und
iii) einer erhöhten Expression des EphA2-Proteins aufweisen.

8. Das Fusionsprotein oder der Expressionsvektor zur Verwendung nach Anspruch 7, wobei die besagten Krebszellen CD20 exprimieren.

9. Das Fusionsprotein oder der Expressionsvektor zur Verwendung nach Anspruch 7 oder 8, wobei die besagten Krebszellen B-Zellen sind.

10. Das Fusionsprotein oder der Expressionsvektor zur Verwendung nach Anspruch 5, wobei das besagte Subjekt ein Mensch ist.

11. Ein *In vitro*-Verfahren für die Identifizierung von Krebszellen, die auf das Fusionsprotein nach den Ansprüchen 1-3 oder den Expressionsvektor nach Anspruch 4 reagieren, das die Bestimmung in den besagten Krebszellen des Vorhandenseins einer Deletion des Ephrinrezeptor A7 (EPHA7)-Gens aufweist, wobei das Erfassen der Deletion des besagten EPHA7-Gens die besagten Krebszellen als Reaktion auf das Fusionsprotein oder den Expressionsvektor identifiziert.

12. Ein *In vitro*-Verfahren für die Identifizierung von Krebszellen, die auf das Fusionsprotein nach den Ansprüchen 1-3 oder den Expressionsvektor nach Anspruch 4 reagieren, das die Bestimmung der Expression des EPhA7-Proteins durch die besagten Krebszellen aufweist, wobei das Erfassen der reduzierten Expression des besagten EPhA7-Proteins die Krebszellen als Reaktion auf das Fusionsprotein oder den Expressionsvektor nach Anspruch 4 identifiziert.

13. Ein In vitro-Verfahren für die Identifizierung von Krebszellen, die auf das Fusionsprotein nach den Ansprüchen 1-3 oder den Expressionsvektor nach Anspruch 4 reagieren, das die Bestimmung der Expression des EPhA2-Proteins durch die besagten Krebszellen aufweist, wobei das Erfassen der erhöhten Expression des besagten EPhA2-Proteins die Krebszellen als Reaktion auf das Fusionsprotein oder den Expressionsvektor identifiziert.

14. Das Fusionsprotein nach den Ansprüchen 1-3 oder der Expressionsvektor nach Anspruch 4 zur Verwendung als Medikament.

## Revendications

1. Protéine de fusion comprenant une chaîne lourde d'immunoglobuline recombinante (Ig) ou partie de celle-ci présentant une région variable (Fv) amino terminal qui se lie spécifiquement à un antigène marqueur de la surface cellulaire du lymphome, dans laquelle ladite chaîne lourde Ig ou la partie de celle-ci est liée à sa terminaison carboxyle par une liaison peptidique à l'aminoacide à amino terminal de la protéine suppresseuse de la tumeur EphA7^{ECD}.

2. Protéine de fusion selon la revendication 1, dans laquelle l'antigène marqueur de la surface cellulaire du lymphome est l'antigène CD20.

3. Protéine de fusion selon la revendication 1 ou 2, dans laquelle ladite protéine suppresseuse de la tumeur EphA7^{ECD} comprend la séquence SÉQ ID n°:02.

4. Vecteur d'expression recombinant comprenant une séquence de nucléotides codant la protéine de la revendication 1 ou 2.

5. Protéine de fusion des revendications 1 à 3 ou vecteur d'expression de la revendication 4 destiné à une utilisation dans une méthode servant à la réduction d'un ou plusieurs symptômes du cancer chez un sujet, dans laquelle ledit cancer est un lymphome.

6. Protéine de fusion ou vecteur d'expression destiné à une utilisation selon la revendication 5, dans laquelle ledit cancer est le lymphome à cellules B, le lymphome folliculaire, le lymphome non hodgkinien, le lymphome de Burkitt ou le lymphome à grandes cellules B.

7. Protéine de fusion ou vecteur d'expression destiné à une utilisation selon la revendication 5, dans laquelle ledit cancer comprend des cellules cancéreuses qui
a. expriment un marqueur de surface des cellules tumorales qui peuvent être liées par la région variable (Fv) à amino terminal d'une immunoglobuline recombinante (Ig) et
b. comprennent une ou plusieurs de
i) la suppression du gène du récepteur de l'éphrine A7 (EPHA7) ;
ii) l'expression réduite de la protéine EphA7, et
iii) l'expression augmentée de la protéine EphA2.

8. Protéine de fusion ou vecteur d'expression destiné à une utilisation selon la revendication 7, dans laquelle lesdites cellules cancéreuses expriment CD20.

9. Protéine de fusion ou vecteur d'expression destiné à une utilisation selon la revendication 7 ou 8, dans laquelle lesdites cellules cancéreuses sont des cellules B.

10. Protéine de fusion ou vecteur d'expression destiné à une utilisation selon la revendication 5, dans laquelle ledit sujet est l'homme.

11. Méthode *in vitro* destinée à identifier les cellules cancéreuses sensibles à la protéine de fusion des revendications 1 à 3 ou au vecteur d'expression de la revendication 4, comprenant la détermination dans lesdites cellules cancéreuses de la présence d'une suppression du gène du récepteur de l'éphrine A7 (EPHA7) dans laquelle la détection de la suppression dudit gène EPHA7 identifie lesdites cellules cancéreuses comme sensibles à la protéine de fusion ou au vecteur d'expression.

12. Méthode *in vitro* destinée à identifier les cellules cancéreuses sensibles à la protéine de fusion des revendications 1 à 3 ou au vecteur d'expression de la revendication 4, comprenant la détermination de l'expression des protéines EphA7 par lesdites cellules cancéreuses, dans laquelle la détection de l'expression réduite de ladite protéine EphA7 identifie lesdites cellules cancéreuses comme sensibles à la protéine de fusion ou au vecteur d'expression de la revendication 4.

13. Méthode *in vitro* destinée à identifier les cellules cancéreuses sensibles à la protéine de fusion des revendications 1 à 3 ou au vecteur d'expression de la revendication 4, comprenant la détermination de l'expression de la protéine EphA2 par lesdites cellules cancéreuses, dans laquelle la détection d'une expression augmentée de ladite protéine EphA2 identifie lesdites cellules cancéreuses comme sensibles à la protéine de fusion ou au vecteur d'expression.

14. Protéine de fusion des revendications 1 à 3 ou vecteur d'expression de la revendication 4 destiné à une utilisation comme médicament.
